# FASCICULE DE BREVET EUROPEEN

(11) **EP 1 129 690 B1**
(45) Date de publication et mention de la délivrance du brevet: **05.01.2005**
(21) Numéro de dépôt: 01400430.3
(22) Date de dépôt: 19.02.2001
(51) Int. Cl.: A61K 7/13, C07D 471/04

(54) **Compositions de teinture des fibres keratiniques contenant des derives d'indolizine cationiques et procede de teinture**
Zusammensetzung zur Färbung von Keratinfasern mit einem Gehalt an kationischen Indolizinederivaten und Färbeverfahren
Keratin fibre dye compositions containing indolizine cationic derivatives and dyeing method

(30) Priorité: 25.02.2000 FR 0002419
(43) Date de publication de la demande: 05.09.2001
(73) Titulaire: L'OREAL, 75008 Paris (FR)
(72) Inventeur: Breton, Philippe, 78150 Le Chesnay (FR); Segala, Fabienne, 75011 Paris (FR); Lagrange, Alain, 77700 Couvray (FR)
(74) Mandataire: Fevrier, Murielle

(56) Documents cités:
- DE-C- 464 481
- GB-A- 2 075 540
- US-A- 3 260 601
- US-A- 3 846 131
- US-A- 4 275 206
- US-A- 4 400 387
- DATABASE CHEMICAL ABSTRACTS [en ligne] STN; access number 74: 88 647, XP002178582 & E. KOCHETKOVA: "cyanine dyes from 2-phenylindolizines" SB NAUCH. TR. VLADIMIR. POLITEKH. INST., no. 7, 1969, pages 146-148,
- D. LIDE ET AL.: "CRC handbook of chemistry and physics, 76th edition" , CRC PRESS , BOCA RATON XP002178581 Page 3-201, compound 7324
- J. HICKMAN ET AL.: "Indolizines. Part V. The synthesis of 3-amino and 3-acetamido-indolizines and their precursors, the 3-azo-,-nitroso-,-nitro-, and -acetyl-indolizines" J. CHEM. SOC., PERKIN TRANS. I, no. 23, 1972, pages 2954-2958, XP001024447
- R. BONNEAU ET AL.: "Synthesis of 3-substituted indolizines from the reaction of chlorocarbenes with 2-vinylpyridine" J. CHEM. SOC., CHEM. COMMUN., vol. 4, 1994, pages 509-510-510, XP001024448

## Description

L'invention a pour objet une composition pour la teinture d'oxydation des fibres kératiniques contenant au moins une base d'oxydation, et à titre de coupleur, au moins un dérivé d'indolizine comportant au moins un groupement cationique Z, Z étant choisi parmi des chaînes aliphatiques quaternisées, des chaînes aliphatiques comportant au moins un cycle saturé quaternisé, et des chaînes aliphatiques comportant au moins un cycle insaturé quaternisé, les procédés de teinture d'oxydation les mettant en oeuvre, ainsi que de nouveaux dérivés d'indolizine cationiques

II est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est-à-dire permettre d'obtenir des écarts de coloration les plus faibles possible tout au long d'une même fibre kératinique, qui peut être en effet différemment sensibilisée (i.e. abîmée) entre sa pointe et sa racine.

L'utilisation de composés de type indazole est connue dans le domaine de la coloration des cheveux. La demande de brevet DE-A-1 492 166 décrit la polycondensation par oxydation de tels composés. La demande de brevet DE-A-2 623 564 décrit l'association d'hydroxy indazoles avec des tétraamino pyrimidines. Le brevet US 4 013 404 décrit certains amino indazoles ainsi que leur utilisation à titre de précurseurs de colorants d'oxydation.

La demanderesse vient maintenant de découvrir, de façon totalement inattendue et surprenante, que les dérivés d'indolizine de formule (I) ci-après définie comportant au moins un groupement cationique Z, Z étant choisi parmi des chaînes aliphatiques quaternisées, des chaînes aliphatiques comportant au moins un cycle saturé quaternisé, et des chaînes aliphatiques comportant au moins un cycle insaturé quaternisé, non seulement conviennent pour une utilisation comme coupleur pour la teinture d'oxydation, mais en outre qu'ils permettent d'obtenir des compositions tinctoriales conduisant à des colorations puissantes, dans une large palette de couleurs, et présentant d'excellentes propriétés de résistances aux différents traitements que peuvent subir les fibres kératiniques.

Cette découverte est à la base de la présente invention.

L'invention a donc pour objet une composition pour la teinture des fibres kératiniques et en particulier des fibres kératiniques humaines telles que les cheveux, caractérisée par le fait qu'elle comprend, dans un milieu approprié pour la teinture :
- à titre de coupleur, au moins un dérivé d'indolizine de formule (I), et/ou au moins l'un de ses sels d'addition avec un acide : 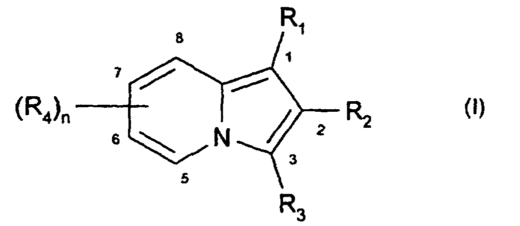 dans laquelle :
   - n est un nombre entier compris entre 0 et 4 inclusivement ;
   - R₁ et R₃, identiques ou différents, représentent un atome d'hydrogène ou d'halogène ; un groupement Z tel que défini ci-après ; un radical alkyle en C₁-C₄ ; alcoxy en C₁-C₄ ; monohydroxyalkyle en C₁-C₄ ; polyhydroxyalkyle en C₂-C₄ ; aminoalkyle en C₁-C₄ ; acétylamino ; acétylaminoalkyle en C₁-C₄ ; alcoxy(C₁-C₄)alkyle en C₁-C₄ ; monoalkyl(C₁-C₄)aminoalkyle en C₁-C₄ ; dialkyl(C₁-C₄)aminoalkyle en C₁-C₄ ; aminocarboxyalkyle en C₁-C₄ ; acétyl alkyl(C₁-C₄)aminoalkyle en C₁-C₄ ; ou un cycle aromatique non substitué ou substitué par un ou plusieurs radicaux choisis parmi un atome d'halogène, un radical alkyle en C₁-C₄, trifluorométhyle, alcoxy en C₁-C₄, amino, et amino mono- ou disubstitué par un radical alkyle en C₁-C₄, par un radical alkyl(C₁-C₄)aminoalkyle en C₁-C₄, par un radical dialkyl(C₁-C₄)aminoalkyle en C₁-C₄, par un radical carboxyle ou par un radical sulfoxy ;
      R₃ peut également représenter un hétérocyclique insaturé à 5 ou 6 chaînons ;
   - R₂ et R₄, identiques ou différents, représentent un atome d'hydrogène ou d'halogène, un groupement Z tel que défini ci-après ; un radical alkyle en C₁-C₄, monohydroxyalkyle en C₁-C₄, polyhydroxyalkyle en C₂-C₄, aminoalkyle en C₁-C₄, monoalkyl(C₁-C₄)aminoalkyle en C₁-C₄, dialkyl(C₁-C₄)aminoalkyle en C₁-C₄, carboxy, cyano, carboxyalkyle en C₁-C₄, nitro, ou un cycle aromatique à 5 ou 6 chaînons non substitué ou substitué par un ou plusieurs radicaux choisis parmi un atome d'halogène, un radical alkyle en C₁-C₄, trifluorométhyle, alcoxy en C₁-C₄, amino, et amino mono- ou disubstitué par un radical alkyle en C₁-C₄, par un radical alkyl(C₁-C₄)aminoalkyle en C₁-C₄, par un radical dialkyl(C₁-C₄)aminoalkyle en C₁-C₄, par un radical carboxyle ou par un radical sulfoxy ;
   - Z est choisi parmi les groupements cationiques insaturés de formules (II) et (III) suivantes, et les groupements cationiques saturés de formule (IV) suivante : 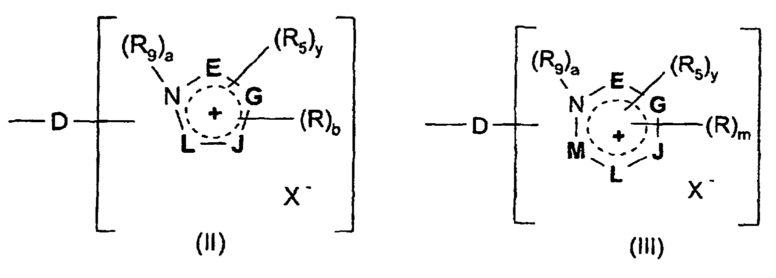 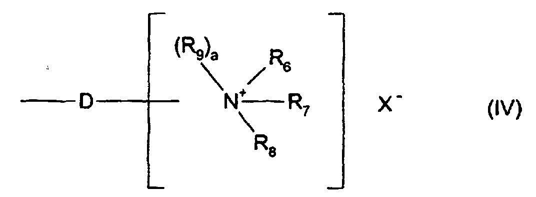 dans lesquelles :
      - D est un bras de liaison qui représente une chaîne alkylène comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C1-C6, et pouvant porter une ou plusieurs fonctions cétone ;
      - les sommets E, G, J, L et M, identiques ou différents, représentent un atome de carbone, d'oxygène, de soufre ou d'azote ;
      - b est un nombre entier compris entre 0 et 4 inclusivement ;
      - m est un nombre entier compris entre 0 et 5 inclusivement ;
      - les radicaux R, identiques ou différents, représentent un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle; un groupement NHR" ou NR"R"' dans lesquels R" et R''', identiques ou différents, représentent un radical alkyle en C₁-C₆, un radical monohydroxyalkyle en C₁-C₆ ou un radical polyhydroxyalkyle en C₂-C₆ ;
   - R₅ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, ou un second groupement Z identique ou différent du premier groupement Z ;
   - R₆, R₇ et R₈, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₆, R₇ et R₈ peuvent également former ensemble, avec l'atome d'azote auquel ils sont rattachés, un cycle saturé à 5 ou 6 chaînons carboné ou contenant un ou plusieurs hétéroatomes tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
      l'un des radicaux R₆, R₇ et R₈ peut également représenter un second groupement Z, identique ou différent du premier groupement Z ;
   - R₉ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
   - a et y sont des nombres entiers égaux à 0 ou 1 ; avec les conditions suivantes :
      - dans les groupements cationiques insaturés de formule (II) :
         - lorsque a = 0, le bras de liaison D est rattaché à l'atome d'azote,
         - lorsque a = 1, le bras de liaison D est rattaché à l'un des sommets E, G, J ou L,
         - y ne peut prendre la valeur 1 que :
            1) lorsque les sommets E, G, J et L représentent simultanément un atome de carbone, et que le radical R₅ est porté par l'atome d'azote du cycle insaturé ; ou bien
            2) lorsqu'au moins un des sommets E, G, J et L représente un atome d'azote sur lequel le radical R₅ est fixé ;
         - lorsque b est supérieur ou égal à 2, deux radicaux R adjacents peuvent également former ensemble, un cycle insaturé à 5 ou 6 chaînons, carboné, ou contenant un ou plusieurs hétéroatomes ;
      - dans les groupements cationiques insaturés de formule (III):
         - lorsque a = 0, le bras de liaison D est rattaché à l'atome d'azote ;
         - lorsque a = 1, le bras de liaison D est rattaché à l'un des sommets E, G, J, L ou M ;
         - y ne peut prendre la valeur 1 que lorsque au moins un des sommets E, G, J, L et M représente un atome divalent, et que le radical R₅ est porté par l'atome d'azote du cycle insaturé ;
         - lorsque m est supérieur ou égal à 2, deux radicaux R adjacents peuvent également former ensemble, un cycle insaturé à 5 ou 6 chaînons, carboné, ou contenant un ou plusieurs hétéroatomes ;
      - dans les groupements cationiques de formule (IV):
         - lorsque a = 0, le bras de liaison D est rattaché à l'atome d'azote portant les radicaux R₆ à R₈,
         - lorsque a = 1, deux des radicaux R₆ à R₈ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
   - X⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
      étant entendu que :
      - au moins un des radicaux R₁ et R₃ représente un atome d'hydrogène ;
      - le nombre de groupement cationique Z est au moins égal à 1 ;
- et au moins une base d'oxydation.

Les colorations obtenues avec la composition de teinture conforme à l'invention, sont de nuances variées, puissantes, peu sélectives et présentent d'excellentes propriétés de résistance à la fois vis-à-vis des agents atmosphériques tels que la lumière et les intempéries et vis-à-vis de la transpiration et des différents traitements que peuvent subir les cheveux (shampooings, déformations permanentes, lumière, transpiration, etc...).

Dans les formules (I), (II), (III) et (IV) ci-dessus les radicaux alkyle et alcoxy peuvent être linéaires ou ramifiés.

Parmi les cycles aromatiques de la formule (I) ci-dessus, on peut notamment citer les cycles phényle, nitrophényle, alkylphényle, alcoxyphényle, polyalkylphényle, et polyalcoxyphényle.

Parmi les hétérocycles insaturés à 5 ou 6 chaînons pouvant être représentés par le radical R₃, on peut notamment citer les cycles pyrrolique, pyridinique, pyrimidinique, imidazolique, pyrazolique, oxazolique, thiazolique, triazolique, pyrazolotriazolique, pyrazoloimidazolique, pyrrolotriazolique, pyrazolopyrimidinique, pyrazolopyridinique, benzoimidazolique, benzoxazolique, benzothiazolique, indolique, indolinique, indolidinique, isoindolidinique, benzotriazolinique, pyrazinique, oxazinique, triazinique, quinolinique, tetrahydroquinolinique, benzoimidazolidinique, et benzopyrimidinique.

Parmi les cycles des groupements insaturés Z de formule (II) ci-dessus, on peut notamment citer à titre d'exemple les cycles pyrrolique, imidazolique, pyrazolique, oxazolique, thiazolique et triazolique. pyrazoltriazolinium, pyrazoloimidazolinium, pyrrolotriazolinium, pyrazolopyrimidinium, pyrazolopyridinium, benzoimidazolinium, benzoxazolinium, benzothiazolinium, indolinium, indolidinium, isoindolinium, indazolinium et benzotriazolinium.

Parmi les cycles des groupements insaturés Z de formule (III) ci-dessus, on peut notamment citer à titre d'exemple les cycles pyridinique, pyrimidinique, pyrazinique, oxazinique et triazinique, quinolinium, tetrahydroquinolinium, benzoimidazolidinium, benzopyrimidinium.

Parmi les dérivés d'indolizine de formule (I), utilisables à titre de coupleur dans la composition tinctoriale conforme à l'invention, on peut notamment citer :
- le N,N-diméthyl-2-phényl-N-(phénylméthyl)-1-indolizine-propanaminium ;
- le N,N,N-triméthyl-2-phényl-1-indolizine-éthanaminium
- le bromure de N,N,N-triméthyl-2-phényl-1-indolizinepropanaminium ;
- le bromure de 4-méthyl-4-[2-(2-phényl-1-indolizinyl)éthyl]-morpholinium ;
- le nitrate de N,N-diméthyl-2-phényl-N-(phénylméthyl)-1-indolizine-propanaminium ;
- le chlorure de N,N-diméthyl-2-phényl-N-(phénylméthyl)-1-indolizine-propanaminium ;
- le méthylsulfate de N,N,N-triméthyl-2-phényl-1-indolizine-éthanaminium ;
- le iodure de triméthyl[2-(2-phényl-1-indolizinyl)éthyl]-ammonium ;
- le bromure de triméthyl[2-(2-phényl-1-indolizinyl)éthyl]-ammonium ;
- le méthyl sulfate de 1-méthyl-2-[2-(7-méthyl-2-phényl-indolizin-3-yl)-éthyl]-pyridinium ;
- le chlorure de 3-méthyl-1-[2-(7-méthyl-2-phényl-indolizin-3-yl)-2-oxo-éthyl]-3H-imidazol-1-ium ;
- le chlorure de 3-méthyl-1-[2-(2-méthyl-indolizin-3-yl-)2-oxo-éthyl]-3H-imidazol-1-ium ;
- le chlorure de 1-{2-[2-(4-méthoxy-phényl)-8-méthyl-indolizin-3-yl]-2-oxo-éthyl}-3-méthyl-3H-imidazol-1-ium ;
- le chlorure de 3-méthyl-1-{2-[8-méthyl-2-(3-nitro-phényl)-indolizin-3-yl]-2-oxo-éthyl}-3H-imidazol-1-ium ;
- le chlorure de 1-{2-[2-(3-nitro-phényl)-indolizin-3-yl]-2-oxo-éthyl}-pyridinium ;
- le chlorure de 1-[2-oxo-2-(2-phényl-indolizin-3-yl)-éthyl]-pyridinium ;
- le chlorure de 1-[2-oxo-2-(2-m-tolyl-indolizin-3-yl)-éthyl]-pyridinium ;
- le chlorure de 1-[2-(8 1-{2-[2-(4-méthoxy-phényl)-8-méthyl-indolizin-3-yl]-2-oxo-éthyl}-2-méthyl-pyridinium ;
- le chlorure de méthyl-2-phényl-indolizin-3-yl)-2-oxo-éthyl] pyridinium ;
- le chlorure de [2-(8-méthyl-2-phényl-indolizin-3-yl)-2-oxo-éthyl]-pyridinium ;
- le chlorure de 2-méthyl-1-{2-[2-(3-nitro-phényl)-indolizin-3-yl]-2-oxo-éthyl}-pyridinium ;
- le chlorure de 2-méthyl-1-[2-oxo-2-(2-phényl-indolizin-3-yl)-éthyl]-pyridinium ;
- le chlorure de 2-méthyl-1-{2-[8-méthyl-2-(3-nitro-phényl)-indolizin-3-yl]-2-oxo-éthyl}-pyridinium ;
- le chlorure de 2-méthyl-1-[2-(8-méthyl-2-phényl-indolizin-3-yl)-2-oxo-éthyl]-pyridinium ;
- le chlorure de 3-méthoxycarbonyl-1-{2-[2-(3-nitro-phényl)-indolizin-3-yl]-2-oxo-éthyl}-pyridinium ;
- le chlorure de 3-méthoxycarbonyl-1-[2-oxo-2-(2-phényl-indolizin-3-yl)-éthyl]-pyridinium ;
- le chlorure de 3-méthoxycarbonyl-1-{2-[2-(4-méthoxy-phényl)-8-méthyl-indolizin-3-yl]-2-oxo-éthyl}-pyridinium ;
- le chlorure de 3-méthoxycarbonyl-1-{2-[8-méthyl-2-(3-nitro-phényl)-indolizin-3-yl]-2-oxo-éthyl}-pyridinium ;
- le chlorure de 3-méthoxycarbonyl-1-[2-(8-méthyl-2-phényl-indolizin-3-yl)-2-oxo-éthyl]-pyridinium ;
et leurs sels d'addition avec un acide.

Le ou les dérivés d'indolizine de formule (I) et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

La nature de la ou des bases d'oxydation utilisées dans la composition tinctoriale conforme à l'invention n'est pas critique. Elles peuvent notamment être choisies parmi les paraphénylènediamines, les bases doubles, les paraaminophénols, les ortho aminophénols et les bases d'oxydation hétérocycliques.

Parmi les paraphénylènediamines utilisables à titre de base d'oxydation dans la composition tinctoriale conforme à l'invention, on peut notamment citer les composés de formule (V) suivante et leurs sels d'addition avec un acide : 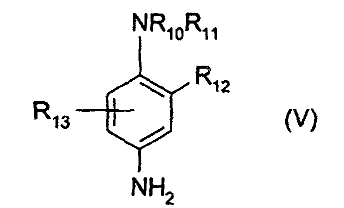 dans laquelle :
- R₁₀ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₁₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₁₂ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₁₃ représente un atome d'hydrogène, d'halogène ou un radical alkyle en C₁-C₄.

Parmi les groupements azotés de la formule (V) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (V) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et-leurs sels d'addition avec un acide.

Parmi les paraphénylènediamines de formule (V) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (III) suivante, et leurs sels d'addition avec un acide : 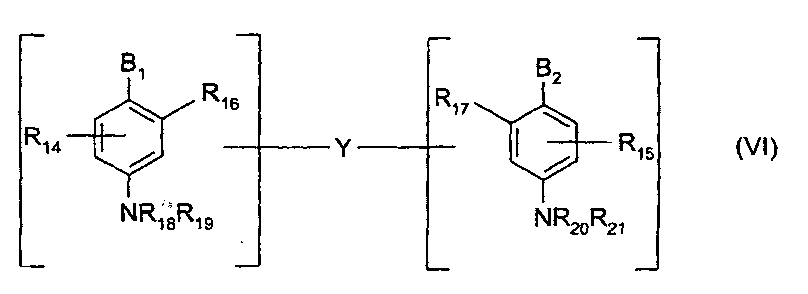 dans laquelle :
- B₁ et B₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₁₄ et R₁₅ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₆, R₁₇, R₁₈, R₁₉, R₂₀ et R₂₁, identiques ou différents, représentent un atome d'hydrogène, un bras de liaison Y ou un radical alkyle en C₁-C₄ ;
étant entendu que les composés de formule (VI) ne comportent qu'un seul bras de liaison Y par molécule.

Parmi les groupements azotés de la formule (VI) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formule (VI) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (VI), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (VII) suivante, et leurs sels d'addition avec un acide : 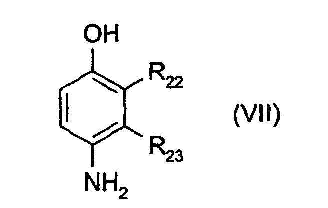 dans laquelle :
- R₂₂ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₂₃ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₂₂ ou R₂₃ représente un atome d'hydrogène.

Parmi les para-aminophénols de formule (VII) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazoio-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1-(β-hydroxyéthyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation représentent de préférence de 0,0005 à 12 % en poids environ du poids total de la composition tinctoriale conforme à l'invention, et encore plus préférentiellement de 0,005 à 6 % en poids environ de ce poids.

La composition tinctoriale conforme à l'invention peut également renfermer un ou plusieurs coupleurs additionnels, (différents des dérivés d'indolizine de formule (I)), et/ou un ou plusieurs colorants directs de façon à faire varier ou enrichir en reflets les nuances obtenues.

Parmi les coupleurs additionnels utilisables dans la composition tinctoriale conforme à l'invention, on peut notamment citer les métaphénylènediamines, les méta-aminophénols, les métadiphénols et les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

Ces coupleurs additionnels sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 2-méthyl-1-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs additionnels représentent de préférence de 0,0001 à 10 % en poids environ du poids total de la composition tinctoriale et encore plus préférentiellement de 0,005 à 5 % en poids environ de ce poids.

Les sels d'addition avec un acide, (dérivés d'indolizine de formule (I), bases d'oxydation et coupleurs additionnels), utilisables dans la composition tinctoriale de l'invention sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol, les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. II peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VIII) suivante : 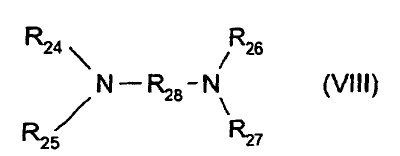 dans laquelle R₂₈ est un reste propylène éventuellement substitué par un groupement hydroxyle ou un radical alkyle en C₁-C₄ ; R₂₄, R₂₅, R₂₆ et R₂₇, identiques ou différents, représentent un atome d'hydrogène, un radical alkyle en C₁-C₄ ou hydroxyalkyle en C₁-C₄.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon invention ne soient pas, ou substantiellement pas, altérées par la ou les adjonctions envisagées.

La composition tinctoriale conforme à l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des dérivés d'indolizine de formule (I) ci-dessus, à titre de coupleur, en association avec au moins une base d'oxydation, pour la teinture d'oxydation des fibres kératiniques et en particulier des fibres kératiniques humaines telles que les cheveux.

Un autre objet de l'invention est un procédé de teinture d'oxydation des fibres kératiniques et en particulier des fibres kératiniques humaines telles que les cheveux mettant en oeuvre la composition tinctoriale telle que définie précédemment.
Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale telle que définie ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides et les enzymes telles que les peroxydases, les laccases, les tyrosynases et les oxydo-réductases parmi lesquelles on peut en particulier mentionner les pyranose oxydases, les glucose oxydases, les glycérol oxydases, les lactates oxydases, les pyruvate oxydases, et les uricases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture à plusieurs compartiments ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Enfin certains dérivés d'indolizine de formule (I) sont nouveaux en soi et constituent à ce titre un autre objet de l'invention.

Ces nouveaux dérivés d'indolizine, et leurs sels d'addition avec un acide répondent à la formule (I) telle que définie précédemment, avec les conditions supplémentaires suivantes :
- Z ne peut pas être choisi parmi les groupements cationiques de formule (IV) telle que définie précédemment ;
- à l'exclusion du bromure de 4-méthyl-4-[2-(2-phényl-1-indolizinyl)-éthyl]-morpholinium.

Les composés exclus sont connus dans le domaine pharmaceutique, voir notamment les brevets US 3 717 644 et US 3 642 807.

Parmi ces nouveaux dérivés d'indolizine conformes à l'invention, on peut plus particulièrement citer :
- le méthyl sulfate de 1-méthyl-2-[2-(7-méthyl-2-phényl-indolizin-3-yl)-éthyl]-pyridinium ;
- le chlorure de 3-méthyl-1-[2-(7-méthyl-2-phényl-indolizin-3-yl)-2-oxo-éthyl]-3H-imidazol-1-ium ;
- le chlorure de 3-méthyl-1-[2-(2-méthyl-indolizin-3-yl-)-2-oxo-éthyl]-3H-imidazol-1-ium ;
- le chlorure de 1-{2-[2-(4-méthoxy-phényl)-8-méthyl-indolizin-3-yl]-2-oxo-éthyl}-3-méthyl-3H-imidazol-1-ium ;
- le chlorure de 3-méthyl-1-{2-[8-méthyl-2-(3-nitro-phényl)-indolizin-3-yl]-2-oxo-éthyl}-3H-imidazol-1-ium ;
- le chlorure de 1-{2-[2-(3-nitro-phényl)-indolizin-3-yl]-2-oxo-éthyl}-pyridinium ;
- le chlorure de 1-[2-oxo-2-(2-phényl-indolizin-3-yl)-éthyl]-pyridinium ;
- le chlorure de 1-[2-oxo-2-(2-m-tolyl-indolizin-3-yl)-éthyl]-pyridinium ;
- le chlorure de 1-[2-(8 1-{2-[2-(4-méthoxy-phényl)-8-méthyl-indolizin-3-yl]-2-oxo-éthyl}-2-méthyl-pyridinium ;
- le chlorure de méthyl-2-phényl-indolizin-3-yl)-2-oxo-éthyl] pyridinium ;
- le chlorure de [2-(8-méthyl-2-phényl-indolizin-3-yl)-2-oxo-éthyl]-pyridinium ;
- le chlorure de 2-méthyl-1-{2-[2-(3-nitro-phényl)-indolizin-3-yl]-2-oxo-éthyl}-pyridinium ;
- le chlorure de 2-méthyl-1-[2-oxo-2-(2-phényl-indolizin-3-yl)-éthyl]-pyridinium ;
- le chlorure de 2-méthyl-1-{2-[8-méthyl-2-(3-nitro-phényl)-indolizin-3-yl]-2-oxo-éthyl}-pyridinium ;
- le chlorure de 2-méthyl-1-[2-(8-méthyl-2-phényl-indolizin-3-yl)-2-oxo-éthyl]-pyridinium ;
- le chlorure de 3-méthoxycarbonyl-1-{2-[2-(3-nitro-phényl)-indolizin-3-yl]-2-oxo-éthyl}-pyridinium ;
- le chlorure de 3-méthoxycarbonyl-1-[2-oxo-2-(2-phényl-indolizin-3-yl)-éthyl]-pyridinium ;
- le chlorure de 3-méthoxycarbonyl-1-{2-[2-(4-méthoxy-phényl)-8-méthylindolizin-3-yl]-2-oxo-éthyl}-pyridinium ;
- le chlorure de 3-méthoxycarbonyl-1-{2-[8-méthyl-2-(3-nitro-phényl)-indolizin-3-yl]-2-oxo-éthyl}-pyridinium ;
- le chlorure de 3-méthoxycarbonyl-1-[2-(8-méthyl-2-phényl-indolizin-3-yl)-2-oxo-éthyl]-pyridinium ;
et leurs sels d'addition avec un acide.

Les sels d'addition avec un acide des composés nouveaux ci-dessus sont choisis parmi ceux définis précédemment pour les composés de formule (I).

Ces composés particuliers peuvent être facilement obtenus selon la méthode de synthèse des indolizines, (Chichibabin, *Ber.* **1927,** 60, 1607 ; Borrows, Holland, Kenyon, *J. Chem. Soc.* **1946,** 1069), consistant à faire réagir une 2-alkyle ou une 2-aralkyle pyridine avec une halocétone pour donner un sel de pyridinium. Le traitement de ce sel par une base, (généralement le bicarbonate de sodium en milieu aqueux) conduit à sa cyclisation en indolizine. D'autres méthodes de synthèse de du noyau indolizinique sont décrites dans la revue de T. Uchida et K. Matsumoto, *Synthesis,* **1976,** 209-236. Ces composés sont ensuite quaternisés selon les méthodes classiques de quaternisation.

Lorsque la synthèse est terminée, ces nouveaux composés conformes à l'invention peuvent, le cas échéant, être récupérés par des méthodes bien connues de l'état de la technique telles que la cristallisation, la distillation.

L'invention a enfin pour objet l'utilisation de ces nouveaux dérivés d'indolizine, et de leurs sels d'addition avec un acide, à titre de coupleur pour la teinture d'oxydation des fibres kératiniques et en particulier des fibres kératiniques humaines telles que les cheveux.

Les exemples qui suivent sont destinés à illustrer l'invention sans pour autant en limiter la portée.

### EXEMPLES DE PREPARATION

### EXEMPLE DE PREPARATION 1 : Synthèse du méthyl sulfate de 1-méthyl-2-[2-(7-méthyl-2-phényl-indolizin-3-yl)-éthyl]-pyridinium

A partir de la 7-méthyl-2-phényl-indolizine, obtenue selon la méthode de synthèse de Chichibabin (Chichibabin *Ber*. **1927,** 60, 1607), on a réalisé une addition catalytique de vinyl pyridine selon la méthode décrite par Pentimalli et Bozzini *Ann*. *Chimica* **1966,** 56, 752. La 7-méthyl-2-phényl-3-(2-pyridin-2-yl-éthyl)-indolizine ainsi obtenue a été traitée par 3 équivalents molaires de diméthylsulfate dans l'acétate d'éthyle au reflux. Après deux heures de réaction, le précipité formé a été filtré, recristallisé de l'alcool isopropylique et le méthyl sulfate de 1-méthyl-2-[2-(7-méthyl-2-phényl-indolizin-3-yl)-éthyl]-pyridinium a été obtenu sous la forme d'un solide beige fondant à une température de 204°C (Kofler).

L'analyse ¹H RMN (DMSO-d₆, 400 MHz) (δ ppm) était la suivante : 2.28 (s, 3H) ; 3.33 (t, 2H) ; 3.37 (s, 3H) ; 3.56 (t, 2H) ; 3.91 (s, 3H) ; 6.35 (s, 1H) ; 6.53 (dd, 1H); 7.22 (m, 1H) ; 7.26 (m, 1H) ; 7.30 (m, 2H) ; 7.35 (m, 2H) ; 7.79 (dd, 1H); 7.84 (ddd, 1H) ; 8.22 (d, 1H) ; 8.30 (ddd, 1H) ; 8.78 (dd, 1H).

### EXEMPLE DE PREPARATION 2 : Synthèse du chlorure de 3-méthyl-1-[2-(7-méthyl-2-phényl-indolizin-3-yl)-2-oxo-éthyl]-3H-imidazol-1-ium

A partir de la 7-méthyl-2-phényl-indolizine, obtenue selon la méthode de synthèse de Chichibabin (Chichibabin *Ber*. **1927,** 60, 1607), on a réalisé une acylation par le chlorure de l'acide chloroacétique dans les conditions suivantes :

A une solution de 5 g (0,024 mole) de 7-méthyl-2-phényl-indolizine et de 2,4 g (0,024 mole) de triéthylamine dans 300 ml de 1,2-dichloroéthane, on a ajouté 2,8 g (0,025 mole) de chlorure de l'acide chloroacétique. Le milieu a été porté au reflux pendant 2H30, puis refroidi à température ambiante. Le milieu réactionnel a été lavé 3 fois par 100 ml d'eau et une fois par 100 ml d'une solution saturée de chlorure de sodium. La phase organique a été séchée sur sulfate de sodium, filtrée, et concentrée à l'évaporateur rotatif. L'huile obtenue a été utilisée sans autre purification à l'étape suivante.

L'huile obtenue a été mise en solution dans 110 ml d'acétate d'éthyle et le mélange a été porté au reflux. On a ajouté 10 g (0,122 mole) de N-méthylimidazole , et le reflux a été poursuivi pendant une heure. Le milieu a ensuite été refroidi à température ambiante, le précipité a été filtré, lavé à l'acétate d'éthyle, puis recristallisé de l'alcool isopropylique. Un solide léger gris très pale fondant à 252°C (Kofler) a été obtenu.

L'analyse ¹H RMN (DMSO-d₆, 400 MHz) (δ ppm) était la suivante : 2.40 (s, 3H) ; 3.89 (s, 3H) ; 5.04 (s, 2H) ; 6.59 (s, 1H) ; 7.02 (dd, 1H) ; 7.48-7.64 (m, 7H) ; 7.72 (dd, 1H) ; 9.14 (dd, 1H) ; 9.71 (d, 1H)

### EXEMPLE DE PREPARATION 3 : Synthèse du chlorure de 3-méthyl-1-[2-(2-méthyl-indolizin-3-yl)-2-oxo-éthyl]-3H-imidazol-1-ium

La 2-méthyl indolizine obtenue selon la synthèse de Chichibabin a été traitée par le chlorure de l'acide chloroacétique comme décrit précédemment ci-dessus à l'exemple de préparation 2. La poudre verte obtenue a été utilisée dans l'étape suivante de quaternisation sans autre purification.

7.9 g de cette poudre verte (0,0383 mole) ont été mis en solution dans 150 ml d'acétate d'éthyle. On ajouté 9,6 g (0,116 mole) de N-méthylimidazole et le milieu a été porté au reflux. Après 3 heures de reflux, on a, à nouveau ajouté 9,6 g (0,116 mole) de N-méthylimidazole. Le reflux a été poursuivi pendant 1 heure, et le milieu réactionnel a été refroidi à température ambiante. Le solide qui a précipité a été filtré, lavé par l'acétate d'éthyle, puis recristallisé de l'alcool isopropylique. Un solide beige fondant à une température supérieure à 260°C (Kofler) a été obtenu.

L'analyse ¹H RMN (DMSO-d₆, 400 MHz) (δ ppm) était la suivante : 2.69 (s, 3H) ; 3.98 (s, 3H) ; 5.90 (s, 2H) ; 6.62 (s, 1H) ; 7.03 (dd, 1H) ; 7.76 (massif, 3H) ; 9.20 (d, 1H) ; 9.79 (d, 1H)

### EXEMPLES DE TEINTURE

### EXEMPLES 1 à 4 DE TEINTURE EN MILIEU ALCALIN

On a préparé les compositions tinctoriales conformes à l'invention suivantes :

| **EXEMPLE** | **1** | **2** | **3** | **4** |
|---|---|---|---|---|
| Méthyl sulfate de 1-méthyl-2-[2-(7-méthyl-2-phényl-indolizin-3-yl)-éthyl]-pyridinium (coupleur de formule (I)) | 3.10⁻³ mole | 3.10⁻³ mole | - | - |
| Chlorure de 3-méthyl-1-[2-(7-méthyl-2-phényl-indolizin-3-yl)-2-oxo-éthyl]-3H-imidazol-1-ium (coupleur de formule (I)) | - | - | 3.10⁻³ mole | 3.10⁻³ mole |
| Paratoluylènediamine (base d'oxydation) | 3.10⁻³ mole | - | 3.10⁻³ mole | - |
| Dichlorhydrate de 3,7-diaminopyrazolopyrimidine (base d'oxydation) | - | 3.10-3 mole | - | 3.10-3 mole |
| Support de teinture commun | (*) | (*) | (*) | (*) |
| Eau déminéralisée q.s.p. | 100 g | 100 g | 100 g | 100 g |

| | | | | |
|---|---|---|---|---|
| (*) : Support de teinture commun : - Ethanol 18 g - Ammoniaque à 20% de NH₃ 10 g - Métabisulfite de sodium 0,205 g - Séquestrant q.s. | | | | |

Au moment de l'emploi, on a mélangé poids pour poids la compositions tinctoriale ci-dessus avec une solution de peroxyde d'hydrogène à 20 volumes (6% en poids) et de pH 3.

Le mélange obtenu présentait un pH d'environ 10 et a été appliqué pendant 30 minutes sur des mèches de cheveux gris naturels à 90 % de blancs. Les mèches ont ensuite été rincées, lavées au shampooing, rincées à nouveau puis séchées.

Les cheveux ont été teints dans une nuance figurant dans le tableau ci-après.

| **EXEMPLE** | **NUANCE OBTENUE** |
|---|---|
| **1** | Blond doré très mat |
| **2** | Blond naturel très cendré |
| **3** | Blond doré légèrement cendré |
| **4** | Blond irisé très cendré |

## Revendications

1. Composition pour la teinture des fibres kératiniques et en particulier des fibres kératiniques humaines telles que les cheveux, **caractérisée par le fait qu'**elle comprend, dans un milieu approprié pour la teinture :
- à titre de coupleur, au moins un dérivé d'indolizine de formule (I), et/ou au moins l'un de ses sels d'addition avec un acide : 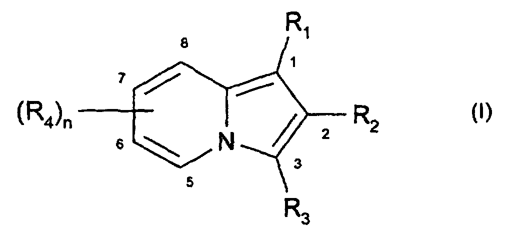 dans laquelle :
- n est un nombre entier compris entre 0 et 4 inclusivement ;
- R₁ et R₃, identiques ou différents, représentent un atome d'hydrogène ou d'halogène ; un groupement Z tel que défini ci-après ; un radical alkyle en C₁-C₄ ; alcoxy en C₁-C₄ ; monohydroxyalkyle en C₁-C₄ ; polyhydroxyalkyle en C₂-C₄ ; aminoalkyle en C₁-C₄ ; acétylamino ; acétylaminoalkyle en C₁-C₄ ; alcoxy(C₁-C₄)alkyle en C₁-C₄ ; monoalkyl(C₁-C₄)aminoalkyle en C₁-C₄ ; dialkyl(C₁-C₄)aminoalkyle en C₁-C₄ ; aminocarboxyalkyle en C₁-C₄ ; acétyl alkyl(C₁-C₄)aminoalkyle en C₁-C₄ ; ou un cycle aromatique non substitué ou substitué par un ou plusieurs radicaux choisis parmi un atome d'halogène, un radical alkyle en C₁-C₄, trifluorométhyle, alcoxy en C₁-C₄, amino, et amino mono- ou disubstitué par un radical alkyle en C₁-C₄, par un radical alkyl(C₁-C₄)aminoalkyle en C₁-C₄, par un radical dialkyl(C₁-C₄)aminoalkyle en C₁-C₄, par un radical carboxyle ou par un radical sulfoxy ;
R₃ peut également représenter un hétérocyclique insaturé à 5 ou 6 chaînons ;
- R₂ et R₄, identiques ou différents, représentent un atome d'hydrogène ou d'halogène, un groupement Z tel que défini ci-après ; un radical alkyle en C₁-C₄, monohydroxyalkyle en C₁-C₄, polyhydroxyalkyle en C₂-C₄, aminoalkyle en C₁-C₄, monoalkyl(C₁-C₄)aminoalkyle en C₁-C₄, dialkyl(C₁-C₄)aminoalkyle en C₁-C₄, carboxy, cyano, carboxyalkyle en C₁-C₄, nitro, ou un cycle aromatique à 5 ou 6 chaînons non substitué ou substitué par un ou plusieurs radicaux choisis parmi un atome d'halogène, un radical alkyle en C₁-C₄, trifluorométhyle, alcoxy en C₁-C₄, amino, et amino mono- ou disubstitué par un radical alkyle en C₁-C₄, par un radical alkyl(C₁-C₄)aminoalkyle en C₁-C₄, par un radical dialkyl(C₁-C₄)aminoalkyle en C₁-C₄, par un radical carboxyle ou par un radical sulfoxy ;
- Z est choisi parmi les groupements cationiques insaturés de formules (II) et (III) suivantes, et les groupements cationiques saturés de formule (IV) suivante : 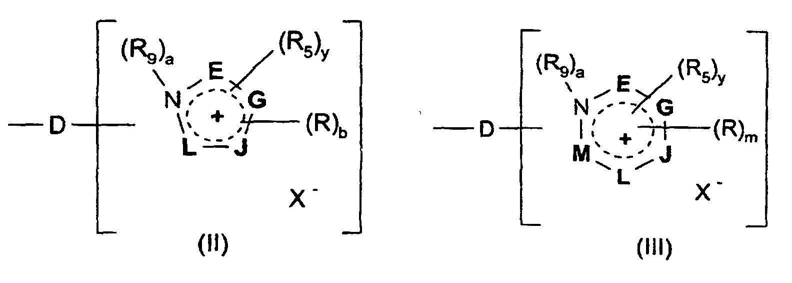 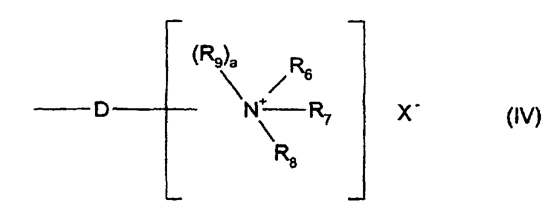 dans lesquelles :
- D est un bras de liaison qui représente une chaîne alkylène comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C1-C6, et pouvant porter une ou plusieurs fonctions cétone ;
- les sommets E, G, J, L et M, identiques ou différents, représentent un atome de carbone, d'oxygène, de soufre ou d'azote ;
- b est un nombre entier compris entre 0 et 4 inclusivement ;
- m est un nombre entier compris entre 0 et 5 inclusivement ;
- les radicaux R, identiques ou différents, représentent un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
- R₅ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, ou un second groupement Z identique ou différent du premier groupement Z ;
- R₆, R₇ et R₈, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₆, R₇ et R₈ peuvent également former ensemble, avec l'atome d'azote auquel ils sont rattachés, un cycle saturé à 5 ou 6 chaînons carboné ou contenant un ou plusieurs hétéroatomes, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
l'un des radicaux R₆, R₇ et R₈ peut également représenter un second groupement Z, identique ou différent du premier groupement Z ;
- R₉ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
- a et y sont des nombres entiers égaux à 0 ou 1 ; avec les conditions suivantes :
- dans les groupements cationiques insaturés de formule (II) :
- lorsque a = 0, le bras de liaison D est rattaché à l'atome d'azote,
- lorsque a = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₅ est porté par l'atome d'azote du cycle insaturé ; ou bien
2) lorsqu'au moins un des sommets E, G, J et L représente un atome d'azote sur lequel le radical R₅ est fixé ;
- lorsque b est supérieur ou égal à 2, deux radicaux R adjacents peuvent également former ensemble, un cycle insaturé à 5 ou 6 chaînons, carboné, ou contenant un ou plusieurs hétéroatomes ;
- dans les groupements cationiques insaturés de formule (III):
- lorsque a = 0, le bras de liaison D est rattaché à l'atome d'azote ;
- lorsque a = 1, le bras de liaison D est rattaché à l'un des sommets E, G, J, L ou M ;
- y ne peut prendre la valeur 1 que lorsque au moins un des sommets E, G, J, L et M représente un atome divalent, et que le radical R₅ est porté par l'atome d'azote du cycle insaturé ;
- lorsque m est supérieur ou égal à 2, deux radicaux R adjacents peuvent également former ensemble, un cycle insaturé à 5 ou 6 chaînons, carboné, ou contenant un ou plusieurs hétéroatomes ;
- dans les groupements cationiques de formule (IV):
- lorsque a = 0, le bras de liaison D est rattaché à l'atome d'azote portant les radicaux R₆ à R₈,
- lorsque a = 1, deux des radicaux R₆ à R₈ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
- X⁻ représente un anion monovalent ou divalent ;
étant entendu que :
- au moins un des radicaux R₁ et R₃ représente un atome d'hydrogène ;
- le nombre de groupement cationique Z est au moins égal à 1 ;
- et au moins une base d'oxydation.

2. Composition selon la revendication 1, **caractérisée par le fait que** les hétérocycles insaturés à 5 ou 6 chaînons pouvant être représentés par le radical R₃ sont choisis parmi les cycles pyrrolique, pyridinique, pyrimidinique, imidazolique, pyrazolique, oxazolique, thiazolique, triazolique, pyrazolotriazolique, pyrazoloimidazolique, pyrrolotriazolique, pyrazolopyrimidinique, pyrazolopyridinique, benzoimidazolique, benzoxazolique, benzothiazolique, indolique, indolinique, indolidinique, isoindolidinique, benzotriazolinique, pyrazinique, oxazinique, triazinique, quinolinique, tetrahydroquinolinique, benzoimidazolidinique, et benzopyrimidinique.

3. Composition selon la revendication 1 ou 2, **caractérisée par le fait que** les cycles des groupements insaturés Z de formule (II) sont choisis parmi les cycles pyrrolique, imidazolique, pyrazolique, oxazolique, thiazolique et triazolique. pyrazoltriazolinium, pyrazoloimidazolinium, pyrrolotriazolinium, pyrazolopyrimidinium, pyrazolopyridinium, benzoimidazolinium, benzoxazolinium, benzothiazolinium, indolinium, indolidinium, isoindolinium, indazolinium et benzotriazolinium.

4. Composition selon la revendication 1 ou 2, **caractérisée par le fait que** les cycles des groupements insaturés Z de formule (III) sont choisis parmi les cycles pyridinique, pyrimidinique, pyrazinique, oxazinique et triazinique, quinolinium, tetrahydroquinolinium, benzoimidazolidinium, benzopyrimidinium.

5. Composition selon l'une quelconque des revendications précédentes, **caractérisée par le fait que** X ⁻ est choisi parmi un atome d'halogène, un hydroxyde, un hydrogènesulfate, ou un alkyl(C₁-C₆)sulfate.

6. Composition selon l'une quelconque des revendications précédentes, **caractérisée par le fait que** les dérivés d'indolizine de formule (I) sont choisis parmi:
- le N,N-diméthyl-2-phényl-N-(phénylméthyl)-1-indolizine-propanaminium ;
- le N,N,N-triméthyl-2-phényl-1-indolizine-éthanaminium
- le bromure de N,N,N-triméthyl-2-phényl-1-indolizinepropanaminium ;
- le bromure de 4-méthyl-4-[2-(2-phényl-1-indolizinyl)éthyl]-morpholinium ;
- le nitrate de N,N-diméthyl-2-phényl-N-(phénylméthyl)-1-indolizine-propanaminium ;
- le chlorure de N,N-diméthyl-2-phényl-N-(phénylméthyl)-1-indolizine-propanaminium ;
- le méthylsulfate de N,N,N-triméthyl-2-phényl-1-indolizine-éthanaminium ;
- le iodure de triméthyl[2-(2-phényl-1-indolizinyl)éthyl]-ammonium ;
- le bromure de triméthyl[2-(2-phényl-1-indolizinyl)éthyl]-ammonium ;
- le méthyl sulfate de 1-méthyl-2-[2-(7-méthyl-2-phényl-indolizin-3-yl)-éthyl]-pyridinium ;
- le chlorure de 3-méthyl-1-[2-(7-méthyl-2-phényl-indolizin-3-yl)-2-oxo-éthyl]-3H-imidazol-1-ium ;
- le chlorure de 3-méthyl-1-[2-(2-méthyl-indolizin-3-yl-)2-oxo-éthyl]-3H-imidazol-1-ium ;
- le chlorure de 1-{2-[2-(4-méthoxy-phényl)-8-méthyl-indolizin-3-yl]-2-oxo-éthyl}-3-méthyl-3H-imidazol-1-ium ;
- le chlorure de 3-méthyl-1-{2-[8-méthyl-2-(3-nitro-phértyl)-indolizin-3-yl]-2-oxo-éthyl}-3H-imidazol-1-ium ;
- le chlorure de 1-{2-[2-(3-nitro-phényl)-indolizin-3-yl]-2-oxo-éthyl}-pyridinium ;
- le chlorure de 1-[2-oxo-2-(2-phényl-indolizin-3-yl)-éthyl]-pyridinium ;
- le chlorure de 1-[2-oxo-2-(2-m-tolyl-indolizin-3-yl)-éthyl]-pyridinium ;
- le chlorure de 1-[2-(8 1-{2-[2-(4-méthoxy-phényl)-8-méthyl-indolizin-3-yl]-2-oxo-éthyl}-2-méthyl-pyridinium ;
- le chlorure de méthyl-2-phényl-indolizin-3-yl)-2-oxo-éthyl] pyridinium ;
- le chlorure de [2-(8-méthyl-2-phényl-indolizin-3-yl)-2-oxo-éthyl]-pyridinium ;
- le chlorure de 2-méthyl-1-{2-[2-(3-nitro-phényl)-indolizin-3-yl]-2-oxo-éthyl}-pyridinium ;
- le chlorure de 2-méthyl-1-[2-oxo-2-(2-phényl-indolizin-3-yl)-éthyl]-pyridinium ;
- le chlorure de 2-méthyl-1-{2-[8-méthyl-2-(3-nitro-phényl)-indolizin-3-yl]-2-oxo-éthyl}-pyridinium ;
- le chlorure de 2-méthyl-1-[2-(8-méthyl-2-phényl-indolizin-3-yl)-2-oxo-éthyl]-pyridinium ;
- le chlorure de 3-méthoxycarbonyl-1-{2-[2-(3-nitro-phényl)-indolizin-3-yl]-2-oxo-éthyl}-pyridinium ;
- le chlorure de 3-méthoxycarbonyl-1-[2-oxo-2-(2-phényl-indolizin-3-yl)-éthyl]-pyridinium ;
- le chlorure de 3-méthoxycarbonyl-1-{2-[2-(4-méthoxy-phényl)-8-méthylindolizin-3-yl]-2-oxo-éthyl}-pyridinium ;
- le chlorure de 3-méthoxycarbonyl-1-{2-[8-méthyl-2-(3-nitro-phényl)-indolizin-3-yl]-2-oxo-éthyl}-pyridinium ;
- le chlorure de 3-méthoxycarbonyl-1-[2-(8-méthyl-2-phényl-indolizin-3-yl)-2-oxo-éthyl]-pyridinium ;
et leurs sels d'addition avec un acide.

7. Composition selon l'une quelconque des revendications précédentes, **caractérisée par le fait que** le ou les dérivés d'indolizine de formule (I) et/ou le ou leurs sels d'addition avec un acide représentent de 0,0005 à 12 % en poids du poids total de la composition tinctoriale.

8. Composition selon l'une quelconque des revendications précédentes, **caractérisée par le fait que** les bases d'oxydation sont choisies parmi les paraphénylènediamines, les bases doubles, les para-aminophénols, les ortho aminophénols et les bases d'oxydation hétérocycliques.

9. Composition selon la revendication 8, **caractérisée par le fait que** les paraphénylènediamines sont choisies parmi les composés de formule (V) suivante et leurs sels d'addition avec un acide : 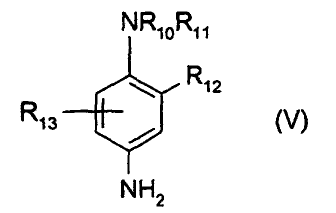 dans laquelle :
- R₁₀ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₁₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₁₂ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₁₃ représente un atome d'hydrogène, d'halogène ou un radical alkyle en C₁-C₄.

10. Composition selon la revendication 9, **caractérisée par le fait que** les paraphénylènediamines sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

11. Composition selon la revendication 8, **caractérisée par le fait que** les bases doubles sont choisies parmi les composés répondant à la formule (III) suivante, et leurs sels d'addition avec un acide : 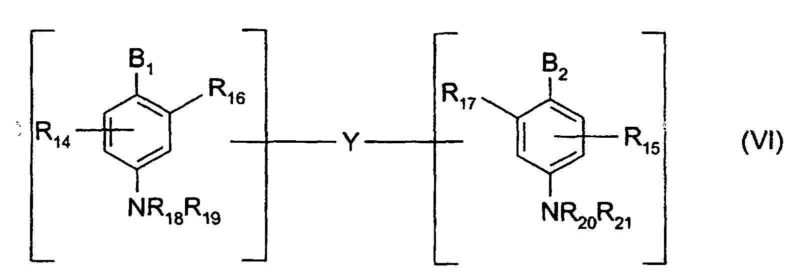 dans laquelle :
- B₁ et B₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₁₄ et R₁₅ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₆, R₁₇, R₁₈, R₁₉, R₂₀ et R₂₁, identiques ou différents, représentent un atome d'hydrogène, un bras de liaison Y ou un radical alkyle en C₁-C₄ ;
étant entendu que les composés de formule (VI) ne comportent qu'un seul bras de liaison Y par molécule.

12. Composition selon la revendication 11, **caractérisée par le fait que** les bases doubles de formule (VI) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

13. Composition selon la revendication 8, **caractérisée par le fait que** les paraaminophénols sont choisis parmi les composés répondant à la formule (VII) suivante, et leurs sels d'addition avec un acide : 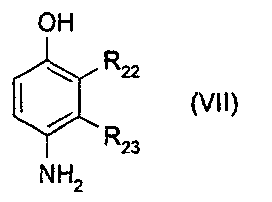 dans laquelle :
- R₂₂ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₂₃ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₂₂ ou R₂₃ représente un atome d'hydrogène.

14. Composition selon la revendication 13, **caractérisée par le fait que** les paraaminophénols sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

15. Composition selon la revendication 8, **caractérisée par le fait que** les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

16. Composition selon la revendication 8, **caractérisée par le fait que** les bases hétérocycliques sont choisies parmi les dérivés pyridiniques, les dérivés pyrimidiniques, les dérivés pyrazoliques, et leurs sels d'addition avec un acide.

17. Composition selon l'une quelconque des revendications précédentes, **caractérisée par le fait que** la ou les bases d'oxydation représentent de 0,0005 à 12 % en poids du poids total de la composition tinctoriale.

18. Composition selon l'une quelconque des revendications précédentes, **caractérisée par le fait qu'**elle renferme un ou plusieurs coupleurs additionnels et/ou un ou plusieurs colorants directs.

19. Composition selon la revendication 18, **caractérisée par le fait que** les coupleurs additionnels sont choisis parmi les métaphénylènediamines, les méta-aminophénols, les métadiphénols et les coupleurs hétérocycliques.

20. Composition selon l'une quelconque des revendications précédentes, **caractérisée par le fait que** le ou les coupleurs additionnels représentent de 0,0001 à 10 % en poids du poids total de la composition tinctoriale.

21. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

22. Utilisation des dérivés d'indolizine de formule (I) tels que définis à l'une quelconque des revendications 1 à 7 et 21, à titre de coupleur, en association avec au moins une base d'oxydation, pour la teinture d'oxydation des fibres kératiniques et en particulier des fibres kératiniques humaines telles que les cheveux.

23. Procédé de teinture d'oxydation des fibres kératiniques et en particulier des fibres kératiniques humaines telles que les cheveux, **caractérisé par le fait que** on applique sur ces fibres au moins une composition tinctoriale telle que définie à l'une quelconque des revendications 1 à 21, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

24. Procédé selon la revendication 23, **caractérisé par le fait que** l'agent oxydant est choisi parmi le peroxyde d'hydrogène, le peroxyde d'urée, les bromates de métaux alcalins, les persels et les enzymes.

25. Procédé selon la revendication 24, **caractérisé par le fait que** les enzymes sont choisies parmi que les peroxydases, les laccases, les tyrosynases et les oxydo-réductases.

26. Dispositif à plusieurs compartiments, dont un premier compartiment renferme une composition tinctoriale telle que définie à l'une quelconque des revendications 1 à 21 et un second compartiment renferme une composition oxydante.

27. Dérivés d'indolizine de formule (I) tels que définis à l'une quelconque des revendications 1 à 7 et 21, dans lesquels Z ne peut pas être choisi parmi les groupements cationiques de formule (IV) telle que définie à la revendication 1 ; et à l'exclusion du bromure de 4-méthyl-4-[2-(2-phényl-1-indolizinyl)-éthyl]-morpholinium.

28. Dérivés selon la revendication 27, **caractérisés par le fait qu'**ils sont choisis parmi :
- le méthyl sulfate de 1-méthyl-2-[2-(7-méthyl-2-phényl-indolizin-3-yl)-éthyl]-pyridinium ;
- le chlorure de 3-méthyl-1-[2-(7-méthyl-2-phényl-indolizin-3-yl)-2-oxo-éthyl]-3H-imidazol-1-ium ;
- le chlorure de 3-méthyl-1-[2-(2-méthyl-indolizin-3-yl-)-2-oxo-éthyl]-3H-imidazol-1-ium ;
- le chlorure de 1-{2-[2-(4-méthoxy-phényl)-8-méthyl-indolizin-3-yl]-2-oxo-éthyl}-3-méthyl-3H-imidazol-1-ium ;
- le chlorure de 3-méthyl-1-{2-[8-méthyl-2-(3-nitro-phényl)-indolizin-3-yl]-2-oxo-éthyl}-3H-imidazol-1-ium ;
- le chlorure de 1-{2-[2-(3-nitro-phényl)-indolizin-3-yl]-2-oxo-éthyl}-pyridinium ;
- le chlorure de 1-[2-oxo-2-(2-phényl-indolizin-3-yl)-éthyl]-pyridinium ;
- le chlorure de 1-[2-oxo-2-(2-m-tolyl-indolizin-3-yl)-éthyl]-pyridinium ;
- le chlorure de 1-[2-(8 1-{2-[2-(4-méthoxy-phényl)-8-méthyl-indolizin-3-yl]-2-oxo-éthyl}-2-méthyl-pyridinium ;
- le chlorure de méthyl-2-phényl-indolizin-3-yl)-2-oxo-éthyl] pyridinium ;
- le chlorure de [2-(8-méthyl-2-phényl-indolizin-3-yl)-2-oxo-éthyl]-pyridinium ;
- le chlorure de 2-méthyl-1-{2-[2-(3-nitro-phényl)-indolizin-3-yl]-2-oxo-éthyl}-pyridinium ;
- le chlorure de 2-méthyl-1-[2-oxo-2-(2-phényl-indolizin-3-yl)-éthyl]-pyridinium ;
- le chlorure de 2-méthyl-1-{2-[8-méthyl-2-(3-nitro-phényl)-indolizin-3-yl]-2-oxo-éthyl}-pyridinium ;
- le chlorure de 2-méthyl-1-[2-(8-méthyl-2-phényl-indolizin-3-yl)-2-oxo-éthyl]-pyridinium ;
- le chlorure de 3-méthoxycarbonyl-1-{2-[2-(3-nitro-phényl)-indolizin-3-yl]-2-oxo-éthyl}-pyridinium ;
- le chlorure de 3-méthoxycarbonyl-1-[2-oxo-2-(2-phényl-indolizin-3-yl)-éthyl]-pyridinium ;
- le chlorure de 3-méthoxycarbonyl-1-{2-[2-(4-méthoxy-phényl)-8-méthyl-indolizin-3-yl]-2-oxo-éthyl}-pyridinium ;
- le chlorure de 3-méthoxycarbonyl-1-{2-[8-méthyl-2-(3-nitro-phényl)-indolizin-3-yl]-2-oxo-éthyl}-pyridinium ;
- le chlorure de 3-méthoxycarbonyl-1-[2-(8-méthyl-2-phényl-indolizin-3-yl)-2-oxo-éthyl]-pyridinium ;
et leurs sels d'addition avec un acide.

29. Utilisation des dérivés d'indolizine selon la revendication 27 ou 28, à titre de coupleur pour la teinture d'oxydation des fibres kératiniques et en particulier des fibres kératiniques humaines telles que les cheveux.

## Claims

1. Composition for dyeing keratin fibres, and in particular human keratin fibres such as the hair, **characterized in that** it comprises, in a medium which is suitable for dyeing:
- as coupler, at least one indolizine derivative of formula (I), and/or at least one of the addition salts thereof with an acid: 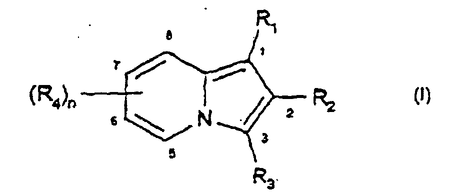 in which:
- n is an integer between 0 and 4 inclusive;
- R₁ and R₃, which may be identical or different, represent a hydrogen or halogen atom; a group Z as defined below; a C₁-C₄ alkyl radical; a C₁-C₄ alkoxy radical; a C₁-C₄ monohydroxyalkyl radical; a C₂-C₄ polyhydroxyalkyl radical; a C₁-C₄ aminoalkyl radical; an acetylamino radical; an acetylamino-(C₁-C₄)alkyl radical; a (C₁-C₄)alkoxy(C₁-C₄)alkyl radical; a mono (C₁-C₄)alkylamino (C₁-C₄)alkyl radical; a di (C₁-C₄)alkylamino(C₁-C₄)alkyl radical; an aminocarboxy(C₁-C₄)alkyl radical; an acetyl(C₁-C₄)alkylamino(C₁-C₄)alkyl radical; or an aromatic ring which is unsubstituted or substituted with one or more radicals chosen from a halogen atom, a C₁-C₄ alkyl radical, a trifluoromethyl radical, a C₁-C₄ alkoxy radical, an amino radical and an amino radical which is mono- or disubstituted with a C₁-C₄ alkyl radical, with a (C₁-C₄) alkylamino (C₁-C₄)alkyl radical, with a di (C₁-C₄)alkylamino (C₁-C₄)alkyl radical, with a carboxyl radical or with a sulphoxy radical; R₃ may also represent a 5- or 6-membered unsaturated heterocycle;
- R₂ and R₄, which may be identical or different, represent a hydrogen or halogen atom, a group Z as defined below; a C₁-C₄ alkyl radical, a C₁-C₄ monohydroxyalkyl radical, a C₂-C₄ polyhydroxyalkyl radical, a C₁-C₄ aminoalkyl radical, a mono(C₁-C₄)-alkylamino (C₁-C₄)alkyl radical, a di(C₁-C₄)alkylamino(C₁-C₄)alkyl radical, a carboxyl radical, a cyano radical, a carboxy(C₁-C₄)alkyl radical, a nitro radical or a 5- or 6-membered aromatic ring which is unsubstituted or substituted with one or more radicals chosen from a halogen atom, a C₁-C₄ alkyl radical, a trifluoromethyl radical, a C₁-C₄ alkoxy radical, an amino radical and an amino radical which is mono- or disubstituted with a C₁-C₄ alkyl radical, with a (C₁-C₄)alkylamino(C₁-C₄)alkyl radical, with a di(C₁-C₄)alkylamino(C₁-C₄)alkyl radical, with a carboxyl radical or with a sulphoxy radical;
- Z is chosen from the following unsaturated cationic groups of formulae (II) and (III) below, and the saturated cationic groups of formula (IV) below: 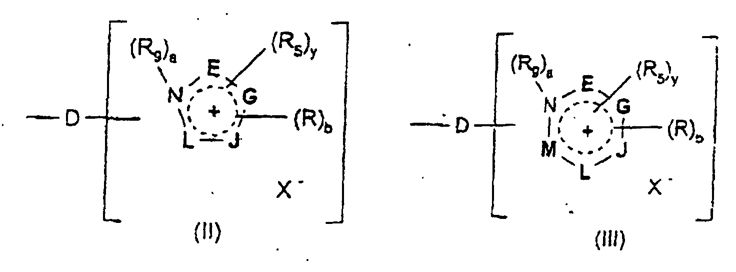 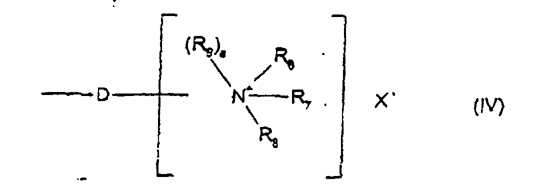 in which:
- D is a linker arm which represents a linear or branched alkylene chain preferably containing from 1 to 14 carbon atoms, which may be interrupted by one or more hetero atoms such as oxygen, sulphur or nitrogen atoms, and which may be substituted with one or more hydroxyl or C₁-C₆ alkoxy radicals, and which may bear one or more ketone functions;
- the ring members E, G, J, L and M, which may be identical or different, represent a carbon, oxygen, sulphur or nitrogen atom;
- b is an integer between 0 and 4 inclusive;
- m is an integer between 0 and 5 inclusive;
- the radicals R, which may be identical or different, represent a second group Z which may be identical to or different from the first group Z, a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a cyano(C₁-C₆)alkyl radical, a C₁-C₆ alkoxy radical, a tri(C₁-C₆)alkylsilane(C₁-C₆)alkyl radical, an amido radical, an aldehydo radical, a carboxyl radical, a (C₁-C₆)alkylcarbonyl radical, a thio radical, a C₁-C₆ thioalkyl radical, a C₁-C₆ alkylthio radical, an amino radical, an amino radical protected with a (C₁-C₆)alkylcarbonyl, carbamyl or C₁-C₆ alkylsulphonyl radical; a group NHR" or NR"R''' in which R" and R''', which may be identical or different, represent a C₁-C₆ alkyl radical, a C₁-C₆ monohydroxyalkyl radical or a C₂-C₆ polyhydroxyalkyl radical;
- R₅ represents a C₁-C₆ alkyl radical, a C₁-C₆ monohydroxyalkyl radical, a C₂-C₆ polyhydroxyalkyl radical, a cyano(C₁-C₆)alkyl radical, a tri(C₁-C₆)-alkylsilane(C₁-C₆)alkyl radical, a (C₁-C₆)alkoxy(C₁-C₆)alkyl radical, a carbamyl (C₁-C₆)alkyl radical, a (C₁-C₆)alkylcarboxy(C₁-C₆)alkyl radical, a benzyl radical or a second group Z which may be identical to or different from the first group Z;
- R₆, R₇ and R₈, which may be identical or different, represent a C₁-C₆ alkyl radical, a C₁-C₆ monohydroxyalkyl radical, a C₂-C₆ polyhydroxyalkyl radical, a (C₁-C₆)alkoxy(C₁-C₆)alkyl radical, a cyano(C₁-C₆)alkyl radical, an aryl radical, a benzyl radical, an amido(C₁-C₆)alkyl radical, a tri(C₁-C₆)alkylsilane(C₁-C₆)alkyl radical or a C₁-C₆ aminoalkyl radical in which the amine is protected with a (C₁-C₆)alkylcarbonyl, carbamyl or C₁-C₆ alkylsulphonyl radical; two of the radicals R₆, R₇ and R₈ can also form, together with the nitrogen atom to which they are attached, a 5- or 6-membered saturated carbon-based ring or a ring containing one or more hetero atoms, it being possible for the said ring to be unsubstituted or substituted with a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a cyano(C₁-C₆)alkyl radical, a C₁-C₆ alkoxy radical, a tri(C₁-C₆)alkylsilane(C₁-C₆)alkyl radical, an amido radical, an aldehydo radical, a carboxyl radical, a keto(C₁-C₆)alkyl radical, a thio radical, a C₁-C₆ thioalkyl radical, a C₁-C₆ alkylthio radical, an amino radical or an amino radical protected with a (C₁-C₆)alkylcarbonyl, carbamyl or C₁-C₆ alkylsulphonyl radical;
one of the radicals R₆, R₇ and R₈ may also represent a second group Z, which may be identical to or different from the first group Z;
- R₉ represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical, a C₁-C₆ aminoalkyl radical in which the amine is protected with a (C₁-C₆)alkylcarbonyl, carbamyl or C₁-C₆ alkylsulphonyl radical; a carboxy(C₁-C₆)alkyl radical; a cyano(C₁-C₆)alkyl radical; a carbamyl(C₁-C₆)alkyl radical; a C₁-C₆ trifluoroalkyl radical; a tri(C₁-C₆)alkylsilane(C₁-C₆)alkyl radical; a sulphonamido(C₁-C₆)alkyl radical; a (C₁-C₆)alkylcarboxy(C₁-C₆)alkyl radical; a (C₁-C₆)alkylsulphinyl(C₁-C₆)alkyl radical; a (C₁-C₆)alkylsulphonyl(C₁-C₆)alkyl radical; a (C₁-C₆)alkylketo(C₁-C₆)alkyl radical; an N-(C₁-C₆)alkylcarbamyl(C₁-C₆)alkyl radical; or an N-(C₁-C₆)alkylsulphonamido(C₁-C₆)alkyl radical;
- a and y are integers equal to 0 or 1; with the following conditions:
- in the unsaturated cationic groups of formula (II) :
- when a = 0, the linker arm D is attached to the nitrogen atom,
- when a = 1, the linker arm D is attached to one of the ring members E, G, J or L,
- y cannot take the value 1 except:
1) when the ring members E, G, J and L simultaneously represent a carbon atom, and when the radical R₅ is borne by the nitrogen atom of the unsaturated ring; or alternatively
2) when at least one of the ring members E, G, J and L represents a nitrogen atom to which the radical R₅ is attached;
- when b is greater than or equal to 2, two adjacent radicals R may also form together an unsaturated 5- or 6-membered carbon-based ring or a ring containing one or more hetero atoms;
- in the unsaturated cationic groups of formula (III) :
- when a = 0, the linker arm D is attached to the nitrogen atom,
- when a = 1, the linker arm D is attached to one of the ring members E, G, J, L or M,
- y cannot take the value 1 except when at least one of the ring members E, G, J, L and M represents a divalent atom, and when the radical R₅ is borne by the nitrogen atom of the unsaturated ring;
- when m is greater than or equal to 2, two adjacent radicals R may also form together an unsaturated 5- or 6-membered carbon-based ring or a ring containing one or more hetero atoms;
- in the cationic groups of formula (IV):
- when a = 0, the linker arm D is attached to the nitrogen atom bearing the radicals R₆ to R₈,
- when a = 1, two of the radicals R₆ to R₈ form, together with the nitrogen atom to which they are attached, a saturated 5- or 6-membered ring as defined above, and the linker arm D is borne by a carbon atom of the said saturated ring;
- X⁻ represents a monovalent or divalent anion;
it being understood that:
- at least one of the radicals R₁ and R₃ represents a hydrogen atom;
- the number of cationic groups Z is at least equal to 1;
- and at least one oxidation base.

2. Composition according to Claim 1, **characterized in that** the unsaturated 5- or 6-membered heterocycles which may be represented by the radical R₃ are chosen from pyrrole, pyridine, pyrimidine, imidazole, pyrazole, oxazole, thiazole, triazole, pyrazolotriazole, pyrazoloimidazole, pyrrolotriazole, pyrazolopyrimidine, pyrazolopyridine, benzimidazole, benzoxazole, benzothiazole, indole, indoline, indolidine, isoindolidine, benzotriazoline, pyrazine, oxazine, triazine, quinoline, tetrahydroquinoline, benzimidazolidine and benzopyrimidine rings.

3. Composition according to Claim 1 or 2, **characterized in that** the rings in the unsaturated groups Z of formula (II) are chosen from pyrrole, imidazole, pyrazole, oxazole, thiazole, triazole, pyrazoltriazolinium, pyrazoloimidazolinium, pyrrolotriazolinium, pyrazolopyrimidinium, pyrazolopyridinium, benzimidazolinium, benzoxazolinium, benzothiazolinium, indolinium, indolidinium, isoindolinium, indazolinium and benzotriazolinium rings.

4. Composition according to Claim 1 or 2, **characterized in that** the rings in the unsaturated groups Z of formula (III) are chosen from pyridine, pyrimidine, pyrazine, oxazine, triazine, quinolinium, tetrahydroquinolinium, benzimidazolidinium and benzopyrimidinium rings.

5. Composition according to any one of the preceding claims, **characterized in that** X⁻ is chosen from a halogen atom, a hydroxide, a hydrogen sulphate and a (C₁-C₆)alkyl sulphate.

6. Composition according to any one of the preceding claims, **characterized in that** the indolizine derivatives of formula (I) are chosen from:
- N,N-dimethyl-2-phenyl-N-(phenylmethyl)-1-indolizinepropanaminium;
- N,N,N-trimethyl-2-phenyl-1-indolizinethanaminium
- N,N,N-trimethyl-2-phenyl-1-indolizinepropanaminium bromide;
- 4-methyl-4-[2-(2-phenyl-1-indolizinyl)ethyl]-morpholinium bromide;
- N,N-dimethyl-2-phenyl-N-(phenylmethyl)-1-indolizinepropanaminium nitrate;
- N,N-dimethyl-2-phenyl-N-(phenylmethyl)-1-indolizinepropanaminium chloride;
- N,N,N-trimethyl-2-phenyl-1-indolizinethanaminium methyl sulphate;
- trimethyl[2-(2-phenyl-1-indolizinyl)ethyl]ammonium iodide;
- trimethyl[2-(2-phenyl-1-indolizinyl)ethyl]ammonium bromide;
- 1-methyl-2-[2-(7-methyl-2-phenylindolizin-3-yl)-ethyl]pyridinium methyl sulphate;
- 3-methyl-1-[2-(7-methyl-2-phenylindolizin-3-yl)-2-oxoethyl]-3H-imidazol-1-ium chloride;
- 3-methyl-1-[2-(2-methylindolizin-3-yl)-2-oxoethyl]-3H-imidazol-1-ium chloride;
- 1-{2-[2-(4-methoxyphenyl)-8-methylindolizin-3-yl]-2-oxoethyl}-3-methyl-3H-imidazol-1-ium chloride;
- 3-methyl-1-{2-[8-methyl-2-(3-nitrophenyl)indolizin-3-yl]-2-oxoethyl}-3H-imidazol-1-ium chloride;
- 1-{2-[2-(3-nitrophenyl)indolizin-3-yl]-2-oxoethyl}pyridinium chloride;
- 1-[2-oxo-2-(2-phenylindolizin-3-yl)ethyl]pyridinium chloride;
- 1-[2-oxo-2-(2-m-tolylindolizin-3-yl)ethyl]pyridinium chloride;
- 1-[2-(8,1-{2-[2-(4-methoxyphenyl)-8-methylindolizin-3-yl]-2-oxoethyl}-2-methylpyridinium chloride;
- 2-phenylindolizin-3-yl)-2-oxoethyl]pyridinium methyl chloride;
- [2-(8-methyl-2-phenylindolizin-3-yl)-2-oxoethyl]-pyridinium chloride;
- 2-methyl-1-{2-[2-(3-nitrophenyl)indolizin-3-yl]-2-oxoethyl}pyridinium chloride;
- 2-methyl-1-[2-oxo-2-(2-phenylindolizin-3-yl)ethyl]-pyridinium chloride;
- 2-methyl-1-{2-[8-methyl-2-(3-nitrophenyl)indolizin-3-yl]-2-oxoethyl}pyridinium chloride;
- 2-methyl-1-[2-(8-methyl-2-phenylindolizin-3-yl)-2-oxoethyl]pyridinium chloride;
- 3-methoxycarbonyl-l-{2-[2-(3-nitrophenyl)indolizin-3-yl]-2-oxoethyl}pyridinium chloride;
- 3-methoxycarbonyl-1-[2-oxo-2-(2-phenylindolizin-3-yl)ethyl]pyridinium chloride;
- 3-methoxycarbonyl-1-{2-[2-(4-methoxyphenyl)-8-methylindolizin-3-yl]-2-oxoethyl}pyridinium chloride;
- 3-methoxycarbonyl-1-{2-[8-methyl-2-(3-nitrophenyl)-indolizin-3-yl]-2-oxoethyl}pyridinium chloride;
- 3-methoxycarbonyl-1-[2-(8-methyl-2-phenylindolizin-3-yl)-2-oxoethyl]pyridinium chloride;
and the addition salts thereof with an acid.

7. Composition according to any one of the preceding claims, **characterized in that** the indolizine derivative(s) of formula (I) and/or the addition salt(s) thereof with an acid represent(s) from 0.0005% to 12% by weight relative to the total weight of the dye composition.

8. Composition according to any one of the preceding claims, **characterized in that** the oxidation bases are chosen from para-phenylenediamines, double bases, para-aminophenols, ortho-aminophenols and heterocyclic oxidation bases.

9. Composition according to Claim 8, **characterized in that** the para-phenylenediamines are chosen from the compounds of formula (V) below, and the addition salts thereof with an acid: 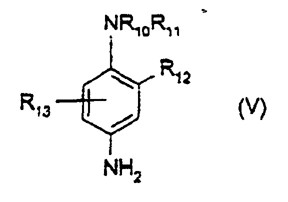 in which:
- R₁₀ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical, a C₁-C₄ alkyl radical substituted with a nitrogenous, phenyl or 4'-aminophenyl group;
- R₁₁ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical, or a C₁-C₄ alkyl radical substituted with a nitrogenous group;
- R₁₂ represents a hydrogen atom, a halogen atom such as a chlorine, bromine, iodine or fluorine atom, a C₁-C₄ alkyl radical, a C₁-C₄ monohydroxyalkyl radical, a C₁-C₄ hydroxyalkoxy radical, an acetylamino(C₁-C₄)alkoxy radical, a C₁-C₄ mesylaminoalkoxy radical or a carbamoylamino(C₁-C₄)alkoxy radical,
- R₁₃ represents a hydrogen or halogen atom or a C₁-C₄ alkyl radical.

10. Composition according to Claim 9, **characterized in that** the para-phenylenediamines are chosen from para-phenylenediamine, para-tolylenediamine, 2-chloro-para-phenylenediamine, 2,3-dimethylpara-phenylenediamine, 2,6-dimethyl-para-phenylenediamine, 2,6-diethyl-para-phenylenediamine, 2,5-dimethyl-para-phenylenediamine, N,N-dimethyl-paraphenylenediamine, N,N-diethyl-para-phenylenediamine, N,N-dipropyl-para-phenylenediamine, 4-amino-N,N-diethyl-3-methylaniline, N,N-bis(β-hydroxyethyl)-paraphenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-paraphenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-paraphenylenediamine, N-phenyl-para-phenylenediamine, 2-β-hydroxyethyloxy-para-phenylenediamine, 2-β-acetylaminoethyloxy-para-phenylenediamine and N-(β-methoxyethyl)-para-phenylenediamine, and the addition salts thereof with an acid.

11. Composition according to Claim 8, **characterized in that** the double bases are chosen from the compounds corresponding to formula (VI) below, and the addition salts thereof with an acid: 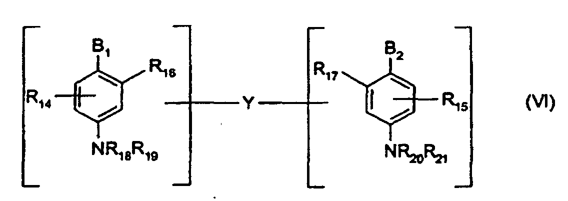 in which:
- B₁ and B₂, which may be identical or different, represent a hydroxyl or -NH₂ radical which may be substituted with a C₁-C₄ alkyl radical or with a linker arm Y;
- the linker arm Y represents a linear or branched alkylene chain containing from 1 to 14 carbon atoms, which may be interrupted or terminated with one or more nitrogenous groups and/or with one or more hetero atoms such as oxygen, sulphur or nitrogen atoms, and optionally substituted with one or more hydroxyl or C₁-C₆ alkoxy radicals;
- R₁₄ and R₁₅ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm Y;
- R₁₆, R₁₇, R₁₈, R₁₉, R₂₀ and R₂₁, which may be identical or different, represent a hydrogen atom, a linker arm Y or a C₁-C₄ alkyl radical;
it being understood that the compounds of formula (VI) comprise only one linker arm Y per molecule.

12. Composition according to Claim 11, **characterized in that** the double bases of formula (VI) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine and 1,8-bis(2,5-diaminophenoxy)-3,5-dioxaoctane, and the addition salts thereof with an acid.

13. Composition according to Claim 8, **characterized in that** the para-aminophenols are chosen from the compounds corresponding to formula (VII) below, and the addition salts thereof with an acid: 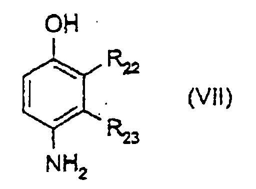 in which:
- R₂₂ represents a hydrogen or halogen atom, a C₁-C₄ alkyl radical, a C₁-C₄ monohydroxyalkyl radical, a (C₁-C₄)alkoxy(C₁-C₄)alkyl radical, a C₁-C₄ aminoalkyl radical or a hydroxy (C₁-C₄)alkylamino (C₁-C₄)alkyl radical,
- R₂₃ represents a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical, a cyano(C₁-C₄)alkyl radical or a (C₁-C₄)alkoxy(C₁-C₄)alkyl radical,
it being understood that at least one of the radicals R₂₂ and R₂₃ represents a hydrogen atom.

14. Composition according to Claim 13, **characterized in that** the para-aminophenols are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

15. Composition according to Claim 8, **characterized in that** the ortho-aminophenols are chosen from 2-aminophenol, 2-amino-5-methylphenol, 2-amino-6-methylphenol and 5-acetamido-2-aminophenol, and the addition salts thereof with an acid.

16. Composition according to Claim 8, **characterized in that** the heterocyclic bases are chosen from pyridine derivatives, pyrimidine derivatives and pyrazole derivatives, and the addition salts thereof with an acid.

17. Composition according to any one of the preceding claims, **characterized in that** the oxidation base(s) represent(s) from 0.0005% to 12% by weight relative to the total weight of the dye composition.

18. Composition according to any one of the preceding claims, **characterized in that** it contains one or more additional couplers and/or one or more direct dyes.

19. Composition according to Claim 18, **characterized in that** the additional couplers are chosen from meta-phenylenediamines, meta-aminophenols, meta-diphenols and heterocyclic couplers.

20. Composition according to any one of the preceding claims, **characterized in that** the additional coupler(s) represent(s) from 0.0001% to 10% relative to the total weight of the dye composition.

21. Composition according to any one of the preceding claims, **characterized in that** the addition salts with an acid are chosen from the hydrochlorides, hydrobromides, sulphates, tartrates, lactates and acetates.

22. Use of the indolizine derivatives of formula (I) as defined in any one of Claims 1 to 7 and 21, as couplers, in combination with at least one oxidation base, for the oxidation dyeing of keratin fibres, and in particular of human keratin fibres such as the hair.

23. Process for the oxidation dyeing of keratin fibres, and in particular of human keratin fibres such as the hair, **characterized in that** at least one dye composition as defined in any one of Claims 1 to 21 is applied to these fibres, and **in that** the colour is developed at acidic, neutral or alkaline pH using an oxidizing agent which is added to the dye composition just at the time of use, or which is present in an oxidizing composition that is applied simultaneously or sequentially in a separate manner.

24. Process according to Claim 23, **characterized in that** the oxidizing agent is chosen from hydrogen peroxide, urea peroxide, alkali metal bromates, persalts and enzymes.

25. Process according to Claim 24, **characterized in that** the enzymes are chosen from peroxidases, laccases, tyrosinases and oxidoreductases.

26. Multi-compartment device, a first compartment of which contains a dye composition as defined in any one of Claims 1 to 21, and a second compartment of which contains an oxidizing composition.

27. Indolizine derivatives of formula (I) as defined in any one of Claims 1 to 7 and 21, in which Z cannot be chosen from the cationic groups of formula (IV) as defined in Claim 1; with the exclusion of 4-methyl-4-[2-(2-phenyl-1-indolizinyl)ethyl]-morpholinium bromide.

28. Derivatives according to Claim 27, **characterized in that** they are chosen from:
- 1-methyl-2-[2-(7-methyl-2-phenylindolizin-3-yl)ethyl]pyridinium methyl sulphate;
- 3-methyl-1-[2-(7-methyl-2-phenylindolizin-3-yl)-2-oxoethyl]-3H-imidazol-1-ium chloride;
- 3-methyl-1-[2-(2-methylindolizin-3-yl)-2-oxoethyl]-3H-imidazol-1-ium chloride;
- 1-{2-[2-(4-methoxyphenyl)-8-methylindolizin-3-yl]-2-oxoethyl}-3-methyl-3H-imidazol-1-ium chloride;
- 3-methyl-1-{2-[8-methyl-2-(3-nitrophenyl)indolizin-3-yl]-2-oxoethyl}-3H-imidazol-1-ium chloride;
- 1-{2-[2-(3-nitrophenyl)indolizin-3-yl]-2-oxoethyl}pyridinium chloride;
- 1-[2-oxo-2-(2-phenylindolizin-3-yl)ethyl]pyridinium chloride;
- 1-[2-oxo-2-(2-m-tolylindolizin-3-yl)ethyl]pyridinium chloride;
- 1-[2-(8,1-{2-[2-(4-methoxyphenyl)-8-methylindolizin-3-yl]-2-oxoethyl}-2-methylpyridinium chloride;
- 2-phenylindolizin-3-yl)-2-oxoethyl]pyridinium methyl chloride;
- [2-(8-methyl-2-phenylindolizin-3-yl)-2-oxoethyl]-pyridinium chloride;
- 2-methyl-1-{2-[2-(3-nitrophenyl)indolizin-3-yl]-2-oxoethyl}pyridinium chloride;
- 2-methyl-1-[2-oxo-2-(2-phenylindolizin-3-yl)ethyl]-pyridinium chloride;
- 2-methyl-1-{2-[8-methyl-2-(3-nitrophenyl)indolizin-3-yl]-2-oxoethyl}pyridinium chloride;
- 2-methyl-1-[2-(8-methyl-2-phenylindolizin-3-yl)-2-oxoethyl]pyridinium chloride;
- 3-methoxycarbonyl-1-{2-[2-(3-nitrophenyl)indolizin-3-yl]-2-oxoethyl}pyridinium chloride;
- 3-methoxycarbonyl-1-[2-oxo-2-(2-phenylindolizin-3-yl)ethyl]pyridinium chloride;
- 3-methoxycarbonyl-1-{2-[2-(4-methoxyphenyl)-8-methylindolizin-3-yl]-2-oxoethyl]pyridinium chloride;
- 3-methoxycarbonyl-1-{2-[8-methyl-2-(3-nitrophenyl)-indolizin-3-yl]-2-oxoethyl}pyridinium chloride;
- 3-methoxycarbonyl-1-[2-(8-methyl-2-phenylindolizin-3-yl)-2-oxoethyl]pyridinium chloride; and the addition salts thereof with an acid.

29. Use of the indolizine derivatives according to Claim 27 or 28, as couplers for the oxidation dyeing of keratin fibres, and in particular of human keratin fibres such as the hair.

## Patentansprüche

1. Zusammensetzung zum Färben von Keratinfasern und insbesondere menschlichen Keratinfasern, wie dem Haar, **dadurch gekennzeichnet, dass** sie in einem zum Färben geeigneten Medium enthält:
- als Kuppler mindestens ein Indolizinderivat der Formel (I) und/oder mindestens eines seiner Additionssalze mit einer Säure: 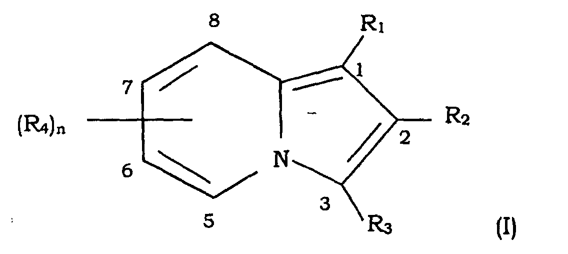 worin bedeuten:
- n 0 oder eine ganze Zahl von 1 bis 4;
- die Gruppen R₁ und R₃, die gleich oder verschieden sind, ein Wasserstoffatom oder ein Halogenatom; eine nachstehend definierte Gruppe Z; C₁₋₄-Alkyl; C₁₋₄-Alkoxy; C₁₋₄-Monohydroxyalkyl; C₂₋₄-Polyhydroxyalkyl; C₁₋₄-Aminoalkyl; Acetylamino; Acetylaminoalkyl(C₁₋₄); C₁₋₄-Alkoxy-C₁₋₄-alkyl; Mono-C₁₋₄-alkylamino-C₁₋₄-alkyl; Di-C₁₋₄-alkylamino-C₁₋₄-alkyl; Aminocarboxyalkyl(C₁₋₄); Acetylalkyl(C₁₋₄)aminoalkyl(C₁₋₄); oder einen aromatischen Ring, der unsubstituiert vorliegt oder mit einer oder mehreren Gruppen substituiert ist, die ausgewählt sind unter: Halogen, C₁₋₄-Alkyl, C₁₋₄-Alkoxy, Amino und Aminogruppen, die mono- oder disubstituiert sind mit C₁₋₄-Alkyl, Alkyl(C₁₋₄)aminoalkyl(C₁₋₄), Dialkyl(C₁₋₄)aminoalkyl(C₁₋₄), Carboxy oder Sulfoxy;
R₃ kann auch einen 5- oder 6-gliedrigen, ungesättigten Heterocyclus bedeuten;
- R₂ und R₄, die gleich oder verschieden sind, ein Wasserstoffatom oder ein Halogenatom, eine nachstehend definierte Gruppe Z; C₁₋₄-Alkyl; C₁₋₄-Monohydroxyalkyl; C₂₋₄-Polyhydroxyalkyl, C₁₋₄-Aminoalkyl, Monoalkyl(C₁₋₄)aminoalkyl(C₁₋₄), Dialkyl(C₁₋₄)aminoalkyl(C₁₋₄), Carboxy, Cyano, Carboxyalkyl(C₁₋₄), Nitro oder einen 5- oder 6-gliedrigen, aromatischen Ring, der unsubstituiert vorliegt oder mit einer oder mehreren Gruppen substituiert ist, die unter einem Halogenatom, C₁₋₄-Alkyl, Trifluormethyl, C₁₋₄-Alkoxy, Amino oder Aminogruppen ausgewählt sind, die mit C₁₋₄-Alkyl, Alkyl(C₁₋₄)aminoalkyl(C₁₋₄), Dialkyl(C₁₋₄)aminoalkyl(C₁₋₄), Carboxy oder Sulfoxy mono- oder disubstituiert sind;
- Z ist unter den ungesättigten kationischen Gruppen der folgenden Formeln (II) und (III) und den gesättigten kationischen Gruppen der folgenden Formel (IV) ausgewählt: 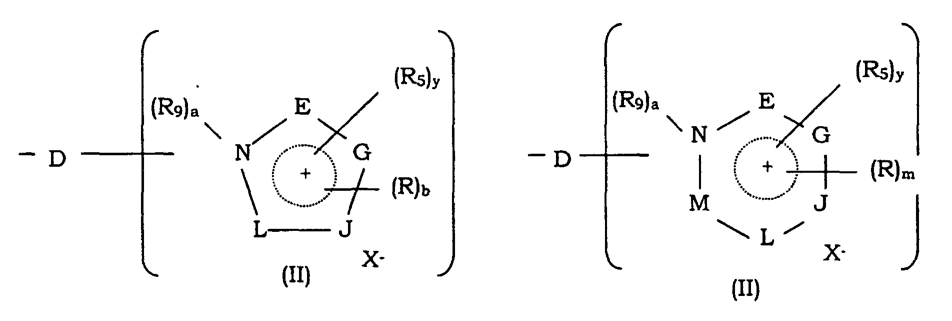 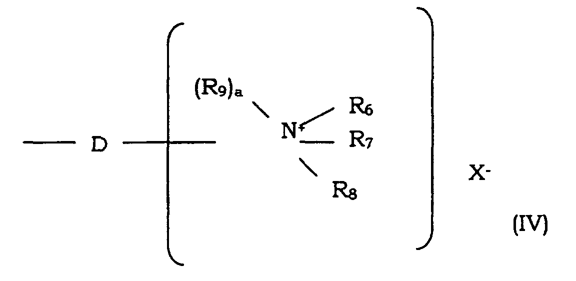 worin bedeuten:
- D eine Verbindungsgruppe, bei der es sich um eine geradkettige oder verzweigte Alkylengruppe handelt, die vorzugsweise 1 bis 14 Kohlenstoffatome enthält und die durch ein oder mehrere Heteroatome, beispielsweise Sauerstoffatome, Schwefelatome oder Stickstoffatome, unterbrochen sein kann und mit einer oder mehreren Hydroxygruppen oder C₁₋₆-Alkoxygruppen substituiert sein kann und die eine oder mehrere Ketofunktionen enthalten kann;
- die Symbole E, G, J, L und M, die gleich oder voneinander verschieden sind, ein Kohlenstoffatom, ein Sauerstoffatom, ein Schwefelatom oder ein Stickstoffatom;
- b 0 oder eine ganze Zahl von 1 bis 4;
- m 0 oder eine ganze Zahl von 1 bis 5;
- die Gruppen R, die gleich oder verschieden sind, eine zweite Gruppe Z, die mit der ersten Gruppe Z identisch oder von ihr verschieden sein kann, ein Halogenatom, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)-silanalkyl(C₁₋₆), Amido, Aldehyde, Carboxy, C₁₋₆-Alkylcarbonyl, Thio, C₁₋₆-Thioalkyl, Alkyl(C₁₋₆)thio, Amino, eine Aminogruppe, die mit einer Alkyl(C₁₋₆)carbonylgruppe, Carbamoylgruppe oder Alkyl(C₁₋₆)sulfonylgruppe geschützt ist; eine Gruppe NHR" oder NR"R"', wobei die Gruppen R" und R"', die gleich oder verschieden sind, eine C₁₋₆-Alkylgruppe, eine C₁₋₄-Monohydroxyalkylgruppe oder eine C₂₋₆-Polyhydroxyalkylgruppe bedeuten;
- R₅ C₁₋₆-Alkyl, C₁₋₆-Monohydroxyalkyl, C₂₋₆-Polyhydroxy, C₁₋₆-Cyanoalkyl, Trialkyl(C₁₋₆)silanalkyl(C₁₋₆), C₁₋₆-Alkoxy-C₁₋₆-alkyl, C₁₋₆-Carbamoylalkyl, Alkyl(C₁₋₆)carboxyalkyl(C₁₋₆), Benzyl, oder eine zweite Gruppe Z, die mit der ersten Gruppe Z identisch oder von ihr verschieden ist;
- die Gruppen R₆, R₇ und R₈, die gleich oder verschieden sind, C₁₋₆-Alkyl, C₁₋₆-Monohydroxyalkyl, C₂₋₆-Polyhydroxyalkyl, C₁₋₆-Alkoxy-C₁₋₆-alkyl, Cyanoalkyl(C₁₋₆), Aryl, Benzyl, C₁₋₆-Aminoalkyl, Trialkyl(C₁₋₆)silanalkyl(C₁₋₆) oder eine Aminoalkylgruppe mit 1 bis 6 Kohlenstoffatomen, deren Aminogruppe mit C₁₋₆-Alkylcarbonyl, Carbamoyl oder C₁₋₆-Alkylsulfonyl geschützt ist, wobei zwei der Gruppen R₆, R₇ und R₈ auch gemeinsam mit dem Stickstoffatom, an das sie gebunden sind, einen gesättigten 5- oder 6-gliedrigen Kohlenstoffring oder Ring, der ein oder mehrere Heteroatome enthält, bilden können, wobei der Ring unsubstituiert vorliegen kann oder mit Halogen, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)silanalkyl(C₁₋₆), Amido, Aldehydo, Carboxy, Ketoalkyl(C₁₋₆), Thio, C₁₋₆-Thioalkyl, C₁₋₆-Alkylthio, Amino, Aminogruppen, die mit Alkyl(C₁₋₆)carbonyl, Carbamoyl oder Alkyl(C₁₋₆)sulfonyl geschützt sind, substituiert ist; wobei eine der Gruppen R₆, R₇ und R₈ auch eine zweite Gruppe Z bedeuten kann, die mit der ersten Gruppe Z identisch oder von ihr verschieden ist;
- R₉ C₁₋₆-Alkyl, C₁₋₆-Monohydroxyalkyl, C₂₋₆-Polyhydroxyalkyl, Aryl, Benzyl, C₁₋₆-Aminoalkyl, C₁₋₆-Aminoalkyl, deren Aminogruppe mit Alkyl(C₁₋₆)carbonyl, Carbamoyl oder Alkyl(C₁₋₆)sulfonyl geschützt ist; Carboxyalkyl(C₁₋₆); Cyanoalkyl(C₁₋₆₎; Carbamoylalkyl(C₁₋₆₎; C₁₋₆-Trifluoralkyl; Trialkyl(C₁₋₆)silanalkyl(C₁₋₆); C₁₋₆-Sulfonamidoalkyl; Alkyl(C₁₋₆)carboxyalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)ketoalkyl(C₁₋₆); N-Alkyl(C₁₋₆)carbamoylalkyl(C₁₋₆); N-Alkyl(C₁₋₆)sulfonamidoalkyl(C₁₋₆);
- a und y bedeuten 0 oder 1; mit den folgenden Maßgaben:
- in den ungesättigten kationischen Gruppen der Formel (II):
- wenn a = 0, ist die Verbindungsgruppe D an das Stickstoffatom gebunden,
- wenn a = 1, ist die Verbindungsgruppe D an einen Ringbestandteil E, G, J oder L gebunden,
- y kann nur den Wert 1 haben, wenn:
1 ) die Ringbestandteile E, G, J und L gleichzeitig Kohlenstoff bedeuten und die Gruppe R₅ von dem Stickstoffatom des ungesättigten Rings getragen wird; oder
2) mindestens ein Ringbestandteil E, G, J und L bedeutet ein Stickstoffatom, an das die Gruppe R₅ gebunden ist;
- wenn b 2 bedeutet oder darüber liegt, können zwei angrenzende Gruppen R auch gemeinsam einen 5- oder 6-gliedrigen ungesättigten Ring auf Kohlenstoffbasis oder Ring mit einem oder mehreren Heteroatomen bilden;
- in den ungesättigten kationischen Gruppen der Formel (III):
- wenn a = 0, ist die Verbindungsgruppe D an das Stickstoffatom gebunden;
- wenn a = 1, ist die Verbindungsgruppe D an einen Ringbestandteil E, G, J, L oder M gebunden;
- y kann nur den Wert 1 annehmen, wenn mindestens ein Ringbestandteil E, G, J, L und M ein zweiwertiges Atom bedeutet und die Gruppe R₅ von dem Stickstoffatom des ungesättigten Rings getragen wird,
- wenn m 2 bedeutet oder darüber liegt, können zwei angrenzende Gruppen R auch gemeinsam einen 5- oder 6-gliedrigen, ungesättigten Ring auf Kohlenstoffbasis oder Ring mit einem oder mehreren Heteroatomen bedeuten;
- in den kationischen Gruppen der Formel (IV):
- wenn a = 0, ist die Verbindungsgruppe D an das Stickstoffatom gebunden, das die Gruppen R₆ bis R₈ trägt,
- wenn a = 1, bilden zwei der Gruppen R₆ bis R₈ gemeinsam mit dem Stickstoffatom, an das sie gebunden sind, einen 5- oder 6-gliedrigen gesättigten Ring, wie er oben definiert wurde, und die Verbindungsgruppe D ist an ein Kohlenstoffatom des gesättigten Rings gebunden;
- X⁻ bedeutet ein einwertiges oder zweiwertiges Anion;
mit der Maßgabe, dass:
- mindestens eine der Gruppen R₁ und R₃ Wasserstoff bedeutet, und
- die Anzahl der kationischen Gruppen Z mindestens 1 ist; und
- mindestens eine Oxidationsbase.

2. Zusammensetzung nach Anspruch 1, **dadurch gekennzeichnet, dass** die 5- oder 6-gliedrigen, ungesättigten Heterocyclen, die die Gruppe R₃ bedeuten kann, unter Pyrrol, Pyridin, Pyrimidin, Imidazol, Pyrazol, Oxazol, Thiazol, Triazol, Pyrazolotriazol, Pyrazoloimidazol, Pyrrolotriazol, Pyrazolopyrimidin, Pyrazolopyridin, Benzoimidazol, Benzoxazol, Benzothiazol, Indol, Indolin, Indolidin, Isoindolidin, Benzotriazolin, Pyrazin, Oxazin, Triazin, Chinolin, Tetrahydrochinolin, Benzoimidazolidin und Benzopyrimidin ausgewählt sind.

3. Zusammensetzung nach Anspruch 1 oder 2, **dadurch gekennzeichnet, dass** die Ringe der ungesättigten Gruppen Z der Formel (II) unter Pyrrol, Imidazol, Pyrazol, Oxazol, Thiazol und Triazol, Pyrazoltriazolinium, Pyrazoloimidazolinium, Pyrrolotriazolinium, Pyrazolopyrimidinium, Pyrazolopyridinium, Benzoimidazolinium, Benzoxazolinium, Benzothiazolinium, Indolinium, Indolidinium, Isoindolinium, Indazolinium und Benzotriazolinium ausgewählt sind.

4. Zusammensetzung nach Anspruch 1 oder 2, **dadurch gekennzeichnet, dass** die Ringe der ungesättigten Gruppen Z der Formel (III) unter Pyridin, Pyrimidin, Pyrazin, Oxazin und Triazin, Chinolinium, Tetrahydrochinolinium, Benzoimidazolidinium und Benzopyrimidinium ausgewählt sind.

5. Zusammensetzung nach einem der vorhergehenden Ansprüche, **dadurch gekennzeichnet, dass** X⁻ unter Halogenatomen, Wasserstoff, Hydrogensulfaten oder C₁₋₆-Alkylsulfaten ausgewählt ist.

6. Zusammensetzung nach einem der vorhergehenden Ansprüche, **dadurch gekennzeichnet, dass** die Indolizinderivate der Formel (I) unter den folgenden Verbindungen ausgewählt sind:
- N,N-Dimethyl-2-phenyl-N-(phenylmethyl)-1-indolizinpropanaminium;
- N,N,N-Trimethyl-2-phenyl-1-indolizin-ethanaminium;
- N,N,N-Trimethyl-2-phenyl-1-indolizin-propanaminiumbromid;
- 4-Methyl-4-[2-(2-phenyl-1-indolizinyl)ethyl]-morpholiniumbromid;
- N,N-Dimethyl-2-phenyl-N-(phenylmethyl)-1-indolizin-propanaminiumnitrat;
- N,N-Dimethyl-2-phenyl-N-(phenylmethyl)-1-indolizin-propanaminiumchlorid;
- N,N,N-Trimethyl-2-phenyl-1-indolizin-ethanaminiummethylsulfat;
- Trimethyl[2-(2-phenyl-1-indolizinyl)ethyl]-ammoniumiodid;
- Trimethyl[2-(2-phenyl-1-indolizinyl)ethyl]-ammoniumbromid;
- 1-Methyl-2-[2-(7-methyl-2-phenyl-indolizin-3-yl)-ethyl]-pyridiniummethylsulfat;
- 3-Methyl-1-[2-(7-methyl-2-phenyl-indolizin-3-yl)-2-oxo-ethyl]-3H-imidazol-1-ium-chlorid;
- 3-Methyl-1-[2-(2-methyl-indolizin-3-yl)2-oxo-ethyl]-3Himidazol-1-ium-chlorid;
- 1-{2-[2-(4-Methoxy-phenyl)-8-methyl-indolizin-3-yl]-2-oxoethyl}-3-methyl-3H-imidazol-1-ium-chlorid;
- 3-Methyl-1-{2-[8-methyl-2-(3-nitro-phenyl)-indolizin-3-yl]-2-oxo-ethyl}-3H-imidazol-1-ium-chlorid;
- 1-{2-[2-(3-Nitro-phenyl)-indolizin-3-yl]-2-oxo-ethyl}pyridiniumchlorid;
- 1-[2-Oxo-2-(2-phenyl-indolizin-3-yl)-ethyl]-pyridiniumchlorid;
- 1-[2-Oxo-2-(2-m-tolyl-indolizin-3-yl)-ethyl]-pyridiniumchlorid;
- 1-[2-(8 1-{2-[2-(4-Methoxy-phenyl)-8-methyl-indolizin-3-yl]-2-oxo-ethyl}-2-methyl-pyridiniumchlorid;
- Methyl-2-phenyl-2-phenyl-indolizin-3-yl)-2-oxo-ethyl]-pyridiniumchlorid;
- [2-(8-Methyl-2-phenyl-indolizin-3-yl)-2-oxo-ethyl]-pyridiniumchlorid;
- 2-Methyl-1-{2-[2-(3-nitro-phenyl)-indolizin-3-yl]-2-oxo-ethyl}pyridiniumchlorid;
- 2-Methyl-1-[2-oxo-2-(2-phenyl-indolizin-3-yl)-ethyl]-pyridiniumchlorid;
- 2-Methyl-1-{2-[8-methyl-2-(3-nitro-phenyl)-indolizin-3-yl]-2-oxo-ethyl}-pyridiniumchlorid;
- 2-Methyl-1-[2-(8-methyl-2-phenyl-indolizin-3-yl)-2-oxo-ethyl]-pyridiniumchlorid;
- 3-Methoxycarbonyl-1-{2-[2-(3-nitro-phenyl)-indolizin-3-yl]-2-oxo-ethyl}-pyridiniumchlorid;
- 3-Methoxycarbonyl-1-[2-oxo-2-(2-phenyl-indolizin-3-yl)-ethyl]-pyridinium-chlorid;
- 3-Methoxycarbonyl-1-{2-[2-(4-methoxy-phenyl)-8-methylindolizin-3-yl]-2-oxo-ethyl)-pyridiniumchlorid;
- 3-Methoxycarbonyl-1-{2-[8-methyl-2-(3-nitro-phenyl)-indolizin-3-yl]-2-oxo-ethyl}-pyridiniumchlorid;
- 3-Methoxycarbonyl-1-[2-(8-methyl-2-phenyl-indolizin-3-yl)-2-oxo-ethyl}-pyridiniumchlorid;
und deren Additionssalzen mit einer Säure.

7. Zusammensetzung nach einem der vorhergehenden Ansprüche, **dadurch gekennzeichnet, dass** das Indolizinderivat der Formel (I) oder die Indolizinderivate der Formel (I) und/oder deren Additionssalz(e) mit einer Säure 0,0005 bis 12 Gew.-% des Gesamtgewichts der Farbmittelzusammensetzung ausmachen.

8. Zusammensetzung nach einem der vorhergehenden Ansprüche, **dadurch gekennzeichnet, dass** die Oxidationsbasen unter den p-Phenylendiaminen, Doppelbasen, p-Aminophenolen, o-Aminophenolen und heterocyclischen Oxidationsbasen ausgewählt sind.

9. Zusammensetzung nach Anspruch 8, **dadurch gekennzeichnet, dass** die p-Phenylendiamine unter den Verbindungen der folgenden Formel (V) und deren Additionssalze mit einer Säure ausgewählt sind: 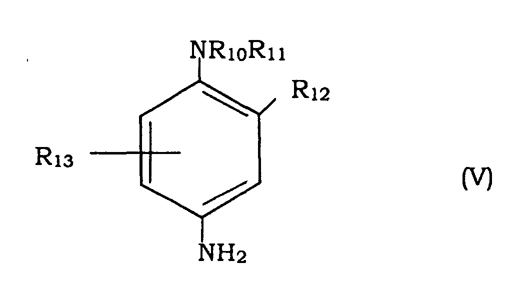 worin bedeuten:
- Rio Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl, eine mit einer stickstoffhaltigen Gruppe substituierte C₁₋₄-Alkylgruppe, Phenyl oder 4'-Aminophenyl;
- R₁₁ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl oder eine mit einer stickstoffhaltigen Gruppe substituierte C₁₋₄-Alkylgruppe;
- R₁₂ Wasserstoff, ein Halogenatom, wie Chlor, Brom, Iod oder Fluor, C₁₋₄-Alkyl, C₁₋₄-Monohydroxyalkyl, C₁₋₄-Hydroxyalkoxy, C₁₋₄-Acetylaminoalkoxy, C₁₋₄-Mesylaminoalkoxy oder C₁₋₄-Carbamoylaminoalkoxy;
- R₁₃ Wasserstoff, Halogen oder C₁₋₄-Alkyl.

10. Zusammensetzung nach Anspruch 9, **dadurch gekennzeichnet, dass** die p-Phenylendiamine unter p-Phenylendiamin, p-Toluylendiamin, 2-Chloro-p-phenylendiamin, 2,3-Dimethyl-p-phenylendiamin, 2,6-Dimethyl-p-phenylendiamin, 2,6-Diethyl-pphenylendiamin, 2,5-Dimethyl-p-phenylendiamin, N,N-Dimethylp-phenylendiamin, N,N-Diethyl-p-phenylendiamin, N,N-Dipropylp-phenylendiamin, 4-Amino-N,N-diethyl-3-methyl-anilin, N,N-Bis(β-hydroxyethyl)-p-phenylendiamin, 4-N,N-Bis(β-hydroxyethyl)amino-2-methyl-anilin, 4-N,N-Bis(β-hydroxyethyl)amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-p-phenylendiamin, N-(β-Methoxyethyl)-p-phenylendiamin und deren Additionssalzen mit einer Säure ausgewählt ist.

11. Zusammensetzung nach Anspruch 8, **dadurch gekennzeichnet, dass** die Doppelbasen unter den Verbindungen der folgenden Formel (V) und deren Additionssalze mit einer Säure ausgewählt sind: 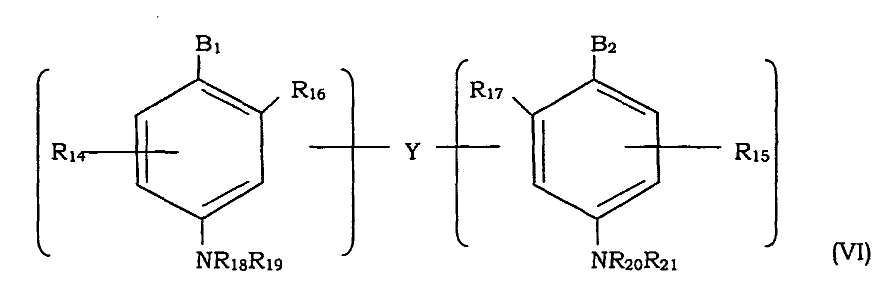 worin bedeuten:
- B₁ und B₂, gleich oder verschieden sind, eine Hydroxygruppe oder die Gruppe -NH₂, die mit einer C₁₋₄-Alkylgruppe oder einer Verbindungsgruppe Y substituiert sein kann;
- die Verbindungsgruppe Y eine geradkettige oder verzweigte Alkylengruppe mit 1 bis 14 Kohlenstoffatomen, die mit einer oder mehreren stickstoffhaltigen Gruppen und/oder einem oder mehreren Heteroatomen, wie Sauerstoff, Schwefel oder Stickstoff, unterbrochen oder abgeschlossen werden und gegebenenfalls mit einer oder mehreren Hydroxygruppen oder C₁₋₆-Alkoxygruppen substituiert sein kann;
- R₁₄ und R₁₅ Wasserstoff, Halogen, C₁₋₄-Alkyl, C₁₋₄-Monohydroxyalkyl, C₂₋₄-Polyhydroxyalkyl, C₁₋₄-Aminoalkyl oder eine Verbindungsgruppe Y; und
- R₁₆, R₁₇, R₁₈, R₁₉, R₂₀ und R₂₁, die gleich oder verschieden sind, ein Wasserstoffatom, eine Verbindungsgruppe Y oder eine C₁₋₄-Alkylgruppe;
mit der Maßgabe, dass die Verbindungen der Formel (VI) nur eine Verbindungsgruppe Y pro Molekül enthalten.

12. Zusammensetzung nach Anspruch 11, **dadurch gekennzeichnet, dass** die Doppelbasen der Formel (VI) unter N,N'-Bis(β-hydroxyethyl)-N,N'-bis(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methyl-aminophenyl)-tetramethylendiamin, N,N'-Bis(ethyl)-N,N'bis(4-amino-3'-methylphenyl)-ethylendiamin, 1,8-Bis(2,5-diaminophenoxy)-3,5-dioxaoctan und deren Additionssalze mit einer Säure ausgewählt sind.

13. Zusammensetzung nach Anspruch 8, **dadurch gekennzeichnet, dass** die p-Aminophenole unter den Verbindungen der folgenden Formel (VII) und deren Additionssalze mit einer Säure ausgewählt sind: 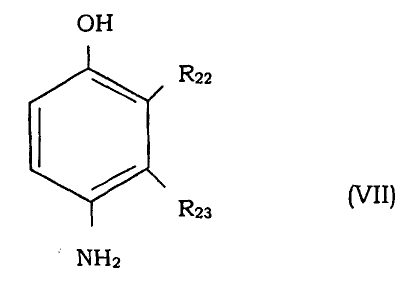 worin bedeuten:
- R₂₂ Wasserstoff, Halogen, C₁₋₄-Alkyl, C₁₋₄-Monohydroxyalkyl, C₁₋₄-Alkoxy-C₁₋₄-alkyl, C₁₋₄-Aminoalkyl oder C₁₋₄-Hydroxyalkyl-C₁₋₄-aminoalkyl;
- R₂₃ Wasserstoff, Halogen, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder C₁₋₄-Alkoxy-C₁₋₄-alkyl,
mit der Maßgabe, dass mindestens eine der Gruppen R₂₂ oder R₂₃ ein Wasserstoffatom bedeutet.

14. Zusammensetzung nach Anspruch 13, **dadurch gekennzeichnet, dass** die p-Aminophenole unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol, 4-Amino-2-fluorphenol und deren Additionssalzen mit einer Säure ausgewählt sind.

15. Zusammensetzung nach Anspruch 8, **dadurch gekennzeichnet, dass** o-Aminophenole unter 2-Aminophenol, 2-Amino-5-methylphenol, 2-Amino-6-methylphenol, 5-Acetamido-2-aminophenol und deren Additionssalzen mit einer Säure ausgewählt sind.

16. Zusammensetzung nach Anspruch 8, **dadurch gekennzeichnet, dass** die heterocyclischen Basen unter den Pyridinderivaten, Pyrimidinderivaten, Pyrazolderivaten und deren Additionssalzen mit einer Säure ausgewählt sind.

17. Zusammensetzung nach einem der vorhergehenden Ansprüche, **dadurch gekennzeichnet, dass** die Oxidationsbase(n) 0,0005 bis 12 Gew.-% des Gesamtgewichts der Zusammensetzung ausmachen.

18. Zusammensetzung nach einem der vorhergehenden Ansprüche, **dadurch gekennzeichnet, dass** sie ferner einen oder mehrere zusätzliche Kuppler und/oder einen oder mehrere Direktfarbstoffe enthält.

19. Zusammensetzung nach Anspruch 18, **dadurch gekennzeichnet, dass** die zusätzlichen Kuppler unter den m-Phenylendiaminen, m-Aminophenolen, m-Dihydroxybenzolen und heterocyclischen Kuppler ausgewählt sind.

20. Zusammensetzung nach einem der vorhergehenden Ansprüche, **dadurch gekennzeichnet, dass** der oder die zusätzliche(n) Kuppler 0,0001 bis 10 Gew.-% des Gesamtgewichts der Farbmittelzusammensetzung ausmachen.

21. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

22. Verwendung von Indolizinderivaten der Formel (I) nach einem der Ansprüche 1 bis 7 und 21 als Kuppler in Kombination mit mindestens einer Oxidationsbase zum oxidativen Färben von Keratinfasern und insbesondere menschlichen Keratinfasern, wie dem Haar.

23. Verfahren zum oxidativen Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 21 aufgebracht wird und die Farbe bei einem sauren, neutralen oder alkalischen pH-Wert mit einem Oxidationsmittel gebildet wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

24. Verfahren nach Anspruch 23, **dadurch gekennzeichnet, dass** das Oxidationsmittel unter Wasserstoffperoxid, Harnstoffperoxid, Alkalimetallbromaten, Salzen von Persäuren und Enzymen ausgewählt ist.

25. Verfahren nach Anspruch 24, **dadurch gekennzeichnet, dass** die Enzyme unter den Peroxidasen, Laccasen, Tyrosinasen und Oxidoreduktasen ausgewählt sind.

26. Vorrichtung mit mehreren Abteilungen, wobei eine Abteilung die Farbmittelzusammensetzung nach einem der Ansprüche 1 bis 21 und eine zweite Abteilung eine oxidierende Zusammensetzung enthält.

27. Indolizinderivate der Formel (I) nach einem der Ansprüche 1 bis 7 und 21, wobei Z nicht unter den kationischen Gruppen der in Anspruch 1 definierten Formel (IV) ausgewählt werden kann und das 4-Methyl-4-[2-(2-phenyl-1-indolizinyl)-ethyl]-morpholiniumbromid ausgenommen ist.

28. Derivate nach Anspruch 27, **dadurch gekennzeichnet, dass** sie ausgewählt sind unter:
- 1-Methyl-2-[2-(7-methyl-2-phenyl-indolizin-3-yl)-ethyl]-pyridinium-methylsulfat;
- 3-Methyl-1-[2-(7-methyl-2-phenyl-indolizin-3-yl)-2-oxo-ethyl]-3H-imidazol-1-ium-chlorid;
- 3-Methyl-1-[2-(2-methyl-indolizin-3-yl)-2-oxo-ethyl]-3Himidazol-1-ium-chlorid;
- 1-{2-[2-(4-Methoxy-phenyl)-8-methyl-indolizin-3-yl]-2-oxoethyl}-3-methyl-3H-imidazol-1-ium-chlorid;
- 3-Methyl-1-{2-[8-methyl-2-(3-nitro-phenyl)-indolizin-3-yl]-2-oxo-ethyl}-3H-imidazol-1-ium-chlorid;
- 1-{2-[2-(3-Nitro-phenyl)-indolizin-3-yl]-2-oxo-ethyl}-pyridiniumchlorid;
- 1-[2-Oxo-2-(2-phenyl-indolizin-3-yl)-ethyl]-pyridinium-chlorid;
- 1-[2-Oxo-2-(2-m-tolyl-indolizin-3-yl)-ethyl]-pyridinium-chlorid;
- 1-[2-(8 1{2-[2-(4-Methoxy-phenyl)-8-methyl-indolizin-3-yl)-2-oxo-ethyl}-2-methyl-pyridinium-chlorid;
- Methyl-2-phenyl-indolizin-3-yl)-2-oxo-ethyl]-pyridiniumchlorid;
- [2-(8-Methyl-2-phenyl-indolizin-3-yl)-2-oxo-ethyl]-pyridiniumchlorid;
- 2-Methyl-1-{2-[2-(3-nitro-phenyl)-indolizin-3-yl)-2-oxo-ethyl}pyridinium-chorid;
- 2-Methyl-1-[2-oxo-2-(2-phenyl-indolizin-3-yl)-ethyl]-pyridinium-chlorid;
- 2-Methyl-1-{2-[8-methyl-2-(3-nitro-phenyl)-indolizin-3-yl]-2-oxo-ethyl}-pyridinium-chlorid;
- 2-Methyl-1-[2-(8-methyl-2-phenyl-indolizin-3-yl)-2-oxo-ethyl]-pyridiniumchlorid;
- 3-Methoxycarbonyl-1-{2-[2-(3-nitro-phenyl)-indolizin-3-yl]-2-oxo-ethyl}-pyridiniumchlorid;
- 3-Methoxycarbonyl-1-[2-oxo-2-(2-phenyl-indolizin-3-yl)-ethyl]-pyridiniumchlorid;
- 3-Methoxycarbonyl-1-{2-[2-(4-methoxy-phenyl)-8-methylindolizin-3-yl]-2-oxo-ethyl}-pyridiniumchlorid;
- 3-Methoxycarbonyl-1-{2-[8-methyl-2-(3-nitro-phenyl)-indolizin-3-yl]-2-oxo-ethyl}-pyridiniumchlorid;
- 3-Methoxycarbonyl-1-[2-(8-methyl-2-phenyl-indolizin-3-yl)-2-oxo-ethyl}-pyridiniumchlorid;
und deren Additionssalzen mit einer Säure.

29. Verwendung von Indolizinderivaten nach Anspruch 27 oder 28 als Kuppler zum oxidativen Färben von Keratinfasern und insbesondere menschlichen Keratinfasern, wie dem Haar.
